(19) Europäisches Patentamt
European Patent Office
Office européen des brevets

(11) **EP 2 387 981 A1**

(12) **EUROPEAN PATENT APPLICATION**
published in accordance with Art. 153(4) EPC

(43) Date of publication:
**23.11.2011 Bulletin 2011/47**

(51) Int Cl.:
*A61F 13/49* *(2006.01)* *A61F 13/15* *(2006.01)*
*A61F 13/53* *(2006.01)* *B32B 5/26* *(2006.01)*
*B32B 27/00* *(2006.01)* *B32B 27/12* *(2006.01)*

(21) Application number: **09838340.9**

(22) Date of filing: **29.06.2009**

(86) International application number:
**PCT/JP2009/061815**

(87) International publication number:
**WO 2010/082373 (22.07.2010 Gazette 2010/29)**

(84) Designated Contracting States:
**AT BE BG CH CY CZ DE DK EE ES FI FR GB GR HR HU IE IS IT LI LT LU LV MC MK MT NL NO PL PT RO SE SI SK TR**

(30) Priority: **19.01.2009 JP 2009009260**

(71) Applicant: **Sumitomo Seika Chemicals Co. Ltd. Kako-gun Hyogo 675-0145 (JP)**

(72) Inventors:
• **KAKIMOTO, Hidenobu**
**Hyogo 672-8076 (JP)**

• **TAKATORI, Junichi**
**Hyogo 672-8076 (JP)**
• **FUKUDOME, Shinya**
**Hyogo 672-8076 (JP)**
• **HANDA, Masayoshi**
**Hyogo 672-8076 (JP)**

(74) Representative: **HOFFMANN EITLE**
**Patent- und Rechtsanwälte**
**Arabellastraße 4**
**81925 München (DE)**

(54) **WATER-ABSORBENT SHEET COMPOSITION**

(57) A water-absorbent sheet composition (51) comprising a structure in which an absorbent layer comprising a water-absorbent resin and an adhesive is sandwiched with two or more sheets of hydrophilic nonwoven fabrics (57), wherein the water-absorbent sheet composition has a structure in which the absorbent layer is fractionated into a primary absorbent layer (53) and a secondary absorbent layer (54), and wherein the water-absorbent sheet composition satisfies (1) the median particle size of the water-absorbent resin (52) used in the primary absorbent layer and the median particle size of the water-absorbent resin (54) used in the secondary absorbent layer are from 100 to 600 $\mu$m; (2) the water absorption rate of saline solution of the water-absorbent resin used in the primary absorbent layer is from 20 to 70 seconds; (3) the water absorption rate of saline solution of the water-absorbent resin used in the secondary absorbent layer is from 1. to 20 seconds; and (4) (the water absorption rate of saline solution of the water-absorbent resin used in the primary absorbent Layer)-( the water absorption rate of saline solution of the water-absorbent resin used in the secondary absorbent layer) is 10 seconds or more. The water-absorbent sheet composition of the present invention exhibits some excellent effects that the water-absorbent sheet composition is capable of accomplishing thinning and avoidance of gel blocking phenomenon and liquid leakage, while obtaining basic properties as a water-absorbent sheet composition at a high level, even for a water-absorbent sheet composition containing a very small amount of pulps.

[Figure 1]

FIG. 1

EP 2 387 981 A1

**Description**

TECHNICAL FIELD

The present invention relates to a thin water-absorbent sheet composition which can be used in the fields of hygienic materials and the like. More specifically, the present invention relates to a water-absorbent sheet composition containing a very small amount of pulp, which can be suitably used in absorbent articles, such as disposable diapers and incontinence pads, having high absorbent properties even when being thin. Furthermore, the present invention relates to an absorbent article obtainable from the water-absorbent sheet composition.

BACKGROUND ART

Body liquid absorbent articles represented by disposable diapers or the like have a structure in which an absorbent material for absorbing a liquid such as a body liquid is sandwiched with a flexible liquid-permeable surface sheet (top sheet) positioned on a side contacting a body and a liquid-impermeable backside sheet (back sheet) positioned on a side opposite to that contacting the body.

Conventionally, there have been increasing demands for thinning and light-weighing of absorbent articles, from the viewpoint of designing property and convenience upon carrying, and efficiency upon distribution. Further, in the recent years, there have been growing needs for so-called eco-friendly intentions, in which resources are effectively utilized so that use of natural materials that require a long time to grow such as trees is avoided as much as possible, from the viewpoint of environmental protection. Conventionally, a method for thinning that is generally carried out in absorbent articles is a method of reducing hydrophilic fibers such as disintegrated pulp of a wood material, which has a role of fixing a water-absorbent resin in an absorbent material, while increasing a water-absorbent resin.

An absorbent material in which a water-absorbent resin is used in a large amount with a lowered proportion of a hydrophilic fiber is preferred in thinning, from the viewpoint of reducing bulky hydrophilic fibers while retaining a liquid. However, when distribution or diffusion of a liquid upon actually using in an absorbent article such as disposable diapers is considered, there is a disadvantage that if a large amount of the water-absorbent resin is formed into a soft gel-like state by absorption, a so-called "gel-blocking phenomenon" takes place, whereby liquid diffusibility is markedly lowered and a liquid permeation rate of the absorbent material is slowed down. This "gel-blocking phenomenon" is a phenomenon in which especially when an absorbent material in which water-absorbent resins are highly densified absorbs a liquid, a water-absorbent resin existing near a surface layer absorbs the liquid to even more densify soft gel that forms near the surface layer, so that a liquid permeation into an internal of an absorbent material is inhibited, thereby making the internal of the water-absorbent resin incapable of efficiently absorbing the liquid.

In view of the above, conventionally, as a means of inhibiting gel blocking phenomenon which takes place by reducing hydrophilic fibers while using a water-absorbent resin in a large amount, for example, proposals such as a method using an absorbent polymer having such properties as specified Saline Flow Conductivity and Performance under Pressure (see Patent Publication 1), and a method using a water-absorbent resin prepared by heat-treating a specified water-absorbent resin precursor with a specified surface crosslinking agent (see Patent Publication 2) have been made.

However, in these methods, the absorption properties as absorbent materials in which water-absorbent resins are used in large amounts are not satisfactory. In addition, there arise some problems that the water-absorbent resin is subjected to be mobile before use or during use because hydrophilic fibers that play a role of fixing the water-absorbent resin are reduced. The absorbent material in which the localization of the absorbent resin takes place is more likely to cause gel-blocking phenomenon.

Further, in an absorbent material of which hydrophilic fibers that contribute to retention of the form are reduced has a lowered strength as an absorbent material, deformation such as twist-bending or tear before or after the absorption of a liquid is likely to take place. In an absorbent material with deformation, liquid diffusibility has markedly lowered, so that abilities inherently owned by the absorbent material cannot be exhibited. In order to try to avoid such phenomena, a ratio of hydrophilic fibers and a water-absorbent resin would be limited, thereby posing limitations in the thinning of an absorbent article.

In view of the above, in recent years, as a next generation style absorbent material which is capable of increasing a content of a water-absorbent resin while using hydrophilic fibers in an absorbent material as little as possible, studies have been widely made on an absorbent laminate that substantially does not contain hydrophilic fibers in an absorbent layer, a water-absorbent sheet composition or the like. For example, included are a method using an absorbent laminate comprising two pieces of nonwoven fabrics, and a reticular layer comprising two, upper and lower layers of hot melt adhesives provided between the nonwoven fabrics, in which the nonwoven fabrics are bonded with the reticular layer (see Patent Publication 3), and the like.

However, in a case where hydrophilic fibers are hardly used, the gel blocking phenomenon as mentioned above are likely to take place. Even in a case where gel blocking phenomenon does not take place, a thing that would serve the role of conventional hydrophilic fibers by which a body fluid such as urea is temporarily subjected to water retention and diffusion of the liquid to an overall absorbent material is lacking, so that a liquid leakage is likely to occur in the absorbent laminate, without being able to sufficiently capture the liquid.

Further, when an adhesive is used for retaining the shape of an absorbent laminate, the surface of an absorbent resin is coated with an adhesive, so that absorbent properties are likely to be lowered. Alternatively, an upper side and a lower side of nonwoven fabrics are firmly adhered with an adhesive to confine an water-absorbent resin in a pouched form or the like, so that the absorption properties inherently owned by the water-absorbent resin are less likely to be exhibited.

When adhesive strength of an absorbent laminate is weakened in order to improve absorption properties, not only a large amount of the absorbent resin is detached upon handling the laminate, thereby making unfavorable economically, but also the laminate is exfoliated due to deficiency in strength, so that there are some possibilities of loss of commercial values. In other words, if adhesion is strengthened, gel blocking phenomenon or liquid leakage occurs, and if adhesion is weakened, the detachment of a water-absorbent resin and the breaking of the laminate take place, so that an absorbent laminate or a water-absorbent sheet composition having satisfactory properties is not obtained.

There is also a method of immobilizing a water-absorbent resin to a substrate without using an adhesive, which is, for example, a method of adhering water-absorbent polymer particles in the process of polymerization to a synthetic fibrous substrate to carry out polymerization on the fibrous substrate (see Patent Publication 4), a method of polymerizing a monomer aqueous composition containing acrylic acid and an acrylic acid salt as main components on a nonwoven fabric substrate by means of electron beam irradiation (see Patent Publication 5), and the like.

In these methods, while the synthetic fibrous substrate is penetrated into the polymer particles to be firmly adhered, there are some disadvantages that it is difficult to complete the polymerization reaction in the substrate, so that unreacted monomers and the like remain in the substrate in large amounts.

In addition, a laminate having a 5-layered structure in which homogeneity is improved and a water-absorbent resin is effectively utilized is disclosed (see Patent Publication 6). The laminate might be effective for a trace amount of liquid (test solution: 0.2 cc); however, not only a total amount of the water-absorbent resin used is small but also a water-absorbent resin in a layer near human body (first absorbent layer) is in a relatively small amount; therefore, when an amount of liquid such as urine or blood is large, the amount of re-wet becomes large, thereby having a disadvantage of increased unpleasant feel.

PRIOR ART PUBLICATIONS

PATENT PUBLICATIONS

Patent Publication 1: Japanese Unexamined Patent Publication No. Hei-9-510889
Patent Publication 2: Japanese Patent Laid-Open Neo. Hei-8-57311
Patent Publication 3: Japanese Patent Laid-Open No. 2000-238161
Patent Publication 4: Japanese Patent Laid-Open No. 2003-11118
Patent Publication 5: Japanese Patent Laid-Open No. Hei-02-048944
Patent Publication 6: Japanese Utility Model Laid-Open No. Hei-6-059039

SUMMARY OF THE INVENTION

PROBLEM TO BE SOLVES BY THE INVENTION

An object of the present invention is to provide a water-absorbent sheet composition which is capable of accomplishing thinning and avoidance of gel blocking phenomenon, irrespective of having a large content of a water-absorbent resin, while obtaining fundamental properties (high strength, fast liquid permeation rate, small amount of re-wet, and small liquid leakage) as a water-absorbent sheet composition at a high level, even for a water-absorbent sheet composition containing a very small amount of pulps.

MEANS TO SOLVE THE PROBLEMS

Specifically, the gist of the present invention relates to:

[1] a water-absorbent sheet composition comprising a structure in which an absorbent layer containing a water-absorbent resin and an adhesive is sandwiched with two or more sheets of hydrophilic nonwoven fabrics, wherein the water-absorbent sheet composition has a structure in which the absorbent layer is fractionated into a primary absorbent layer and a secondary absorbent layer, and wherein the water-absorbent sheet composition satisfies:

(1) the median particle size of the water-absorbent resin used in the primary absorbent layer and the median particle size of the water-absorbent resin used in the secondary absorbent layer are from 100 to 600 $\mu$m;
(2) the water absorption rate of saline solution of the water-absorbent resin used in the primary absorbent layer is from 20 to 70 seconds;
(3) the water absorption rate of saline solution of the water-absorbent resin used in the secondary absorbent layer is from 1 to 20 seconds; and
(4) (the water absorption rate of saline solution of the water-absorbent resin used in the primary absorbent layer)-(the water absorption rate of saline solution of the water-absorbent resin used in the secondary absorbent layer) is 10 seconds or more; and

[2] an absorbent article comprising the water-absorbent sheet composition as defined in the above [1], sandwiched between a liquid-permeable sheet and a liquid-impermeable sheet.

EFFECTS OF THE INVENTION

The water-absorbent sheet composition of the present invention exhibits some excellent effects that the water-absorbent sheet composition is capable of accomplishing thinning and avoidance of gel blocking phenomenon and liquid leakage, while obtaining basic properties as a water-absorbent sheet composition at a high level, even for a water-absorbent sheet composition containing a very small amount of pulps.

BRIEF DESCRIPTION OF THE DRAWING

[Figure 1] Figure 1 is an enlarged cross-sectional view schematically showing one example of a structure of a water-absorbent sheet composition of the present invention.
[Figure 2] Figure 2 is a schematic view of a measurement apparatus used for measuring an initial water absorption rate and an effective amount of water absorbed of a water-absorbent resin.
[Figure 3] Figure 3 is a schematic view showing arrangements of a water-absorbent sheet composition and an acrylic plate, for evaluating strength of the water-absorbent sheet composition.
[Figure 4] Figure 4 is a schematic view of an apparatus used for carrying out a slope leakage test.
[Figure 5] Figure 5 is an electron micrograph showing a structure of particles of a water-absorbent resin A.
[Figure 6] Figure 6 is an electron micrograph showing a structure of particles of a water-absorbent resin C.
[Figure 7] Figure 7 is an electron micrograph showing a structure of particles of a water-absorbent resin D.
[Figure 8] Figure 8 is an electron micrograph showing a structure of particles of a water-absorbent resin E.

MODES FOR TARRYING OUT THE INVENTION

The water-absorbent sheet composition of the present invention has a structure in which an absorbent layer containing a water-absorbent resin and an adhesive is sandwiched with two or more sheets of hydrophilic nonwoven fabrics, wherein the water-absorbent sheet composition has a structure in which the absorbent layer is fractionated into a primary absorbent layer and a secondary absorbent layer, and one of the features of the water-absorbent sheet composition is in that the water-absorbent resin used in the primary absorbent layer and the water-absorbent resin used in the secondary absorbent layer have particular medium particle sizes and particular water absorption capacities. By having the above structure, a water absorbent resin having a slowed initial water absorption rate is used in the primary absorbent layer, thereby accomplishing both of prevention of gel blocking phenomenon at an early stage of urination, and efficient water permeation to the secondary absorbent layer, and a water-absorbent resin having a very fast water absorption rate in the secondary absorbent layer, thereby accomplishing prevention of liquid leakage. Further, the absorbent layers do not substantially contain hydrophilic fibers such as pulps that contribute to fixation of a water-absorbent resin in the absorbent layers, and shape retention of the absorbent layer, and whereby the water-absorbent sheet composition obtained is thin and has high-performance, with a very small amount of pulps used.
As the kinds of the water-absorbent resins, commercially available water-absorbent resins can be used. For example, the water absorbent resin includes hydrolysates of starch-acrylonitrile graft copolymers, neutralized products of starch-acrylic acid graft polymers, saponified products of vinyl acetate-acrylic acid ester copolymers, partially neutralized products of polyacrylic acid, and the like. Among them, the partially neutralized products of polyacrylic acids are preferred, from the viewpoint of production amount, production costs, water absorbency of product, and the like. Methods for synthesizing partially neutralized products of polyacrylic acid include reversed phase suspension polymerization method and aqueous solution polymerization method. Among them, the water-absorbent resins obtained according to reversed phase suspension polymerization method are more preferably used, from the viewpoint of excellent flowability of the resulting particles, smaller amounts of fine powder, high water-absorbent properties, such as water absorption capacity and water-absorption rate.

More specifically, an embodiment where a water-absorbent resin used in at least one of the absorbent layers is a water-absorbent resin obtained by reversed phase suspension polymerization method is preferred, an embodiment where a water-absorbent resin used in a secondary absorbent layer is a water-absorbent resin obtained by reversed phase suspension polymerization method is more preferred, and an embodiment where both the water-absorbent resins used in the primary absorbent layer and the secondary absorbent layer are water-absorbent resins obtained by reversed phase suspension polymerization method is even more preferred.

The partially neutralized product of a polyacrylic acid has a degree of neutralization of preferably 50% by mol or more, and even more preferably from 70 to 90% by mol, from the viewpoint of increasing osmotic pressure of the water-absorbent resin, thereby increasing water absorption properties.

The water-absorbent resin is contained, in a total amount of a primary absorbent layer and a secondary absorbent layer, in the water-absorbent sheet composition of from 100 to 1000 g per one square meter of the water-absorbent sheet composition, i.e. 100 to 1000 $g/m^2$, preferably from 200 to 800 g per one square meter of the water-absorbent sheet composition, i.e. 200 to 800 $g/m^2$, more preferably from 220 to 700 $g/m^2$, even more preferably from 250 to 600 $g/m^2$, and still even more preferably from 270 to 550 $g/m^2$, from the viewpoint of obtaining sufficient water-absorption ability even when a water-absorbent sheet composition of the present invention is used for an absorbent article. The water-absorbent resin is contained in an amount of preferably 100 $g/m^2$ or more, from the viewpoint of exhibiting sufficient water absorption ability as a water-absorbent sheet composition, thereby suppressing re-wetting, and the water-absorbent resin is contained in an amount of preferably 1000 $g/m^2$ or less, from the viewpoint of suppressing the generation of gel blocking phenomenon, exhibiting liquid diffusibility as a water-absorbent sheet composition, and further improving a liquid permeation rate.

The resin ratio (mass ratio) of the primary absorbent layer/secondary absorbent layer is preferably within the range of from primary absorbent layer/secondary absorbent layer = 98/2 to 50/50, more preferably with the range of from primary absorbent layer/secondary absorbent layer = 98/2 to 60/40, more preferably within the range of from primary absorbent layer/secondary absorbent layer = 98/2 to 70/30, even more preferably within the range of from primary absorbent layer/secondary absorbent layer = 95/5 to 80/20, and still even more preferably within the range of from primary absorbent layer/secondary absorbent layer = 95/5 to 85/15. The ratio of the primary absorbent layer is preferably 98 or less, from the viewpoint of sufficiently exhibiting water absorbency of a secondary absorbent layer, thereby preventing liquid leakage, and the ratio of the primary absorbent layer is preferably 50 or more, from the viewpoint of increasing dry feel of the primary absorbent layer after liquid absorption, thereby reducing re-wet.

The absorption properties of the water-absorbent sheet composition of the present invention is influenced by the water absorption properties of the water-absorbent resin used. Therefore, it is preferable that the water-absorbent resin to be used in the present invention is those selected with optimal ranges in water absorption properties such as water absorption capacity (expressed by indices such as effective amount of water absorbed and water-retention capacity), and water absorption rate of the water-absorbent resin, by taking the constitution of each component of the water-absorbent sheet composition or the like into consideration.

In the present specification, the water absorption rate of the water-absorbent resin is evaluated as a water absorption rate of saline solution. The water-absorbent resin used in the primary absorbent layer has a water-absorption rate of saline solution of from 20 to 70 s, more preferably from 25 to 60 s, and even more preferably from 30 to 55 s, from the viewpoint of speeding up the permeation rate of the water-absorbent sheet composition of the present invention, thereby avoiding residence of a liquid in the primary absorbent layer, to increase dry feel to the skin upon use in an absorbent article. The water-absorbent resin used in the secondary absorbent layer has a water-absorption rate of saline solution of from 1 to 20 s, more preferably from 2 to 15 s, and even more preferably from 3 to 10 s, from the viewpoint of reducing leakage at slope of a water-absorbent sheet composition of the present invention, thereby preventing unpleasant feel caused by liquid leakage upon use in an absorbent article. The water-absorption rate of the water-absorbent resin as used herein is a value obtainable by a measurement method described in Examples set forth below.

In the composition of the present invention, it is preferable that there is a positive difference in values between the water absorption rate of saline solution of a water-absorbent resin used in the primary absorbent layer and the rate of that used in the secondary absorbent layer. The greater the difference therebetween, effects of avoiding the stagnation of a liquid in the primary absorbent layer, to thereby increase dry feel, and effects of preventing a liquid leakage are even more strongly exhibited. Specifically, (the water absorption rate of saline solution of a water-absorbent resin used in the primary absorbent layer) - (the water absorption rate of saline solution of a water-absorbent resin used in the secondary absorbent layer) is preferably 10 seconds or more, more preferably 15 seconds or more, and even more preferably 20 seconds or more.

The water-absorbent resin used in the present invention has a median particle size of from 100 to 600 $\mu$m, more preferably from 150 to 550 $\mu$m, and even more preferably from 200 to 500 $\mu$m, irrespective of whether the absorbent layer is primary or secondary. It is preferable that the water-absorbent resin has a median particle size of 100 $\mu$m or more, from the viewpoint of avoiding the use of fine powder which has a worsened flowability as a powder and is likely to generate a gel blocking phenomenon upon water absorption, thereby enhancing workability during the production of a water-absorbent sheet composition, and basic properties of a water-absorbent sheet composition. It is preferable that the water-absorbent resin has a median particle size of 600 $\mu$m or less, from the viewpoint of reducing the rugged feel of the water-absorbent sheet composition, thereby improving texture.

In general, those water-absorbent resins having a large median particle size are likely to have slower water absorption rate, and those having a small median particle size are likely to have a faster water absorption rate, It is possible that a resin having a water absorption rate that is fast in the same level as that used in a secondary absorbent layer in the present invention is obtained, for example, when a median particle size in a conventional water-absorbent resin is made to a size of less than 100 $\mu$m. However, when a median particle size of a water-absorbent resin is made small to a size of less than 100 $\mu$m, flowability as powder become greatly worsened, so that there arise some problems in lowering in productivity due to worsening of working environment due to powdered state, and scattering and detachment of a water-absorbent resin from a hydrophilic nonwoven fabric. Further, when the amount of fine powder of a water-absorbent resin increases, the gel block phenomenon as mentioned above is more likely to take place, thereby leading to lowering of the absorption properties of the water-absorbent sheet composition. Besides, an adhesive effect is lowered probably because a water-absorbent resin having a small median particle size is more likely to cover over an adhesive, thereby making it likely to lower the strength of a water-absorbent sheet composition.

The present inventors have found that in order to avoid these problems, it is important to use a water-absorbent resin having an appropriate median particle size and a fast water absorption rate, especially in a secondary absorbent layer. In order to obtain a water-absorbent resin as described above, it is preferable that a specified method for producing a water-absorbent resin is used, for example, it is preferable to use an aqueous solution polymerization method in which continuous bubbles are introduced by foaming during polymerization, or to use a reverse phased polymerization method using a specified emulsifying agent, among which the latter method is more preferred, from the viewpoint of obtaining high water absorption properties and a fast water absorption rate is stably obtained. As a specified emulsifying agent, a nonionic surfactant having an appropriate hydrophilicity is preferably used, and a water-absorbent resin from a reversed phase suspension polymerization using them is usually obtained in a spherical or granular form, and an agglomerated form thereof. The resin having the form mentioned above is preferably used from the viewpoint that not only pulverization is hardly needed, but also a water-absorbent sheet composition has excellent flowability as a powder, and has excellent operability during the production, or the like.

On the other hand, it is preferable that the water-absorbent resin used in the primary absorbent layer has, in addition to the water absorption rate of saline solution within the range mentioned above, a specified initial water absorption rate and a specified effective amount of water absorbed. The initial water absorption rate is expressed as an amount of water absorbed of a liquid per second in the water absorption period of from 0 to 30 s, and the initial water absorption rate is preferably 0.35 mL/s or less, from the viewpoint of suppressing the generation of a gel blocking phenomenon in an initial stage of the liquid permeation, thereby accelerating liquid diffusion in a primary absorbent layer and efficiently forwarding the liquid into a secondary absorbent layer. The initial water absorption rate is more preferably from 0.05 to 0.30 mL/s, and even more preferably from 0.10 to 0.25 mL/s. The initial water absorption rate is more preferably 0.05 mL/s or more, from the viewpoint of obtaining dry feel to skin in an initial stage of liquid permeation while diffusing the liquid.

The effective amount of water absorbed of the water-absorbent resin used in the primary absorbent layer, in terms of an effective amount of water absorbed for saline solution, is preferably 45 mL/g or more, more preferably from 50 to 80 mL/g, and even more preferably from 55 to 70 mL/g. The water-absorbent resin has an effective amount of water absorbed of preferably 45 mL/g or more, from the viewpoint of allowing a water-absorbent resin to absorb more liquid, and reducing re-wet, thereby obtaining dry feel, and the water-absorbent resin has an effective amount of water absorbed of preferably 80 mL/g or less, from the viewpoint of providing appropriate crosslinking of the water-absorbent resin, thereby keeping gel strength upon absorption and preventing gel blocking.

As mentioned above, the water absorption rate of the water-absorbent resin is likely to be slowed if a median particle size becomes large. However, with regard to the initial water absorption rate (mL/s), this effect is small even when a median particle size is made large in a conventional water-absorbent resin, so that in a conventional water-absorbent resin, even if a median particle size is made to a size of, for example, 600 $\mu$m or more, an initial water absorption rate needed in the present invention is unlikely to be obtained. Moreover, if a median particle size of a water-absorbent resin is made as large as 600 $\mu$m or more, it is undesirable because feel in the water-absorbent sheet composition would be worsened. In view of the above, in a case where a water-absorbent resin having a particular range of particle sizes is used, as a method of controlling an initial water absorption rate of a primary absorbent layer, for example, a method for producing a water-absorbent resin comprising increasing a crosslinking density of a water-absorbent resin with a crosslinking agent capable of reacting with a carboxyl group, or homogeneously coating a surface of a water-absorbent resin with a hydrophobic additive, or carrying out reversed phase suspension polymerization using a specified emulsifying agent, or the like is considered.

However, if a crosslinking density of a water-absorbent resin is increased with a crosslinking agent capable of reacting with a carboxyl group, a specified initial water absorption rate might be satisfied but at the same time an effective amount of water absorbed (absorption capacity) of the water-absorbent resin is lowered, so that it is difficult to obtain a water-absorbent resin satisfying both a specified initial water absorption rate and an effective amount of water absorbed.

Accordingly, in the present invention, a water-absorbent resin used in a primary absorbent layer is more preferably those in which a hydrophobic additive is homogeneously coated on a surface of a water-absorbent resin, and those produced according to reversed phase suspension polymerization using a specified emulsifying agent, from the viewpoint of facilitation in the production of a water-absorbent resin having both a specified initial water absorption rate and a specified effective amount of water absorbed, among which the latter method is even more preferred from the viewpoint of high water absorption properties. As a specified emulsifying agent, a nonionic surfactant having an appropriate hydrophobicity is preferably used, and a water-absorbent resin from a reversed phase suspension polymerization using them is obtained usually in a spherical or American football-shaped form, or an agglomerated form thereof. The resin having the above form is preferably used from the viewpoint that pulverization is hardly needed, and has excellent flowability as powder and excellent operability during the production of a water-absorbent sheet composition.

Here, the effective amount of water absorbed of the water-absorbent resin used in a secondary absorbent layer is not particularly limited, and the effective amount of water absorbed is preferably 30 mL/g or more, and even more preferably 45 mL/g or more.

The effective amount of water absorbed of the water-absorbent resin as used herein is a value obtainable by a measurement method described in Examples set forth below.

The adhesive includes, for example, rubber adhesives such as natural rubbers, butyl rubbers, and polyisoprene; styrenic elastomer adhesives such as styrene-isoprene block copolymers (SIS), styrenebutadiene block copolymers (SBS), styrene-isobutylene block copolymers (SIBS), and styrene-ethylene-butylene-styrene block copolymers (SEBS); ethylene-vinyl acetate copolymer (EVA) adhesives; ethylene-acrylic acid derivative copolymer adhesives such as ethylene-ethyl acrylate copolymer (EEA), and ethylene-butyl acrylate copolymer (EBA); ethylene-acrylic acid copolymer (EAA) adhesives; polyamide adhesives such as copolymer nylons and dimer acids-based polyamides; polyolefin adhesives such as polyethylenes, polypropylenes, atactic polypropylenes, and copolymeric polyolefins; polyester adhesives such as polyethylene terephthalate (PET), polybutylene terephthalate (PBT), and copolymeric polyesters; and acrylic adhesives, and these adhesives may be used together in two or more kinds. In the present invention, the ethylene-vinyl acetate copolymer adhesives, the styrenic elastomer adhesives, the polyolefinic adhesives, and the polyester adhesives are preferred, from the viewpoint of high adhesive strength, thereby making it possible to prevent exfoliation of a hydrophilic nonwoven fabric and scattering of the water-absorbent resin in the water-absorbent sheet composition.

The adhesive has a melting temperature or a softening point of preferably from 60° to 180°C, and more preferably from 70° to 150°C, from the viewpoint of sufficiently fixing a water-absorbent resin to a nonwoven fabric, and at the same time preventing thermal deterioration or deformation of the nonwoven fabric. Here, in the water-absorbent sheet composition of the present invention, in the process of producing a water-absorbent sheet composition, after melting, the adhesive is adhered to a nonwoven fabric or a hydrophilic resin in a solid state by cooling the molten adhesive.

The adhesive in the water-absorbent sheet composition is contained in an amount preferably in the range of from 0.05 to 2.0 times, more preferably in the range of from 0.08 to 1.5 times, and even more preferably in the range of from 0.1 to 1.0 time the amount of the water-absorbent resin contained (mass basis). It is preferable that the adhesive is contained in an amount of 0.05 times or more, from the viewpoint of having sufficient adhesion, thereby preventing exfoliation of the hydrophilic nonwoven fabrics themselves or scattering of the water-absorbent resin, and increasing strength of a water-absorbent sheet composition. It is preferable that the adhesive is contained in an amount of 2.0 times or less, from the viewpoint of avoiding the inhibition of the swelling of the water-absorbent resin due to too strong adhesion to each other, thereby improving a permeation rate or liquid leakage of a water-absorbent sheet composition.

The absorbent layer contains a water-absorbent resin and an adhesive, and the absorbent layer is formed by, for example, evenly dispersing a mixed powder of a water-absorbent resin and an adhesive on a hydrophilic nonwoven fabric, and further overlaying with a breathable fractionating layer as occasion demands, and subjecting overlaid layers to heating, if necessary, heating under pressure near a melting temperature of the adhesive.

The hydrophilic nonwoven fabric is not particularly limited, as long as the hydrophilic nonwoven fabric is a known nonwoven fabric in the field of art. The hydrophilic nonwoven fabric includes nonwoven fabrics made of polyolefin fibers such as polyethylene (PE) and polypropylene (PP); polyester fibers such as polyethylene terephthalate (PET), polytrimethylene terephthalate (PTT), and polyethylene naphthalate (PEN); polyamide fibers such as nylon; rayon fibers, and other synthetic fibers; nonwoven fabrics produced by mixing cotton, silk, hemp, pulp (cellulose) fibers, or the like, from the viewpoint of liquid permeability, flexibility and strength upon forming into a sheet composition, and the hydrophilic nonwoven fabric may be a mixture of two or more kinds of fibers. In addition, its surface may be subjected to a hydrophilic treatment according to a known method, as occasion demands. The nonwoven fabric made of synthetic fibers is preferably used, from the viewpoint of increasing the strength of the water-absorbent sheet composition, and especially at least one member selected from the group consisting of rayon fibers, polyolefin fibers, polyester fibers, and mixtures thereof is preferred. The hydrophilic nonwoven fabric made of synthetic fibers may contain pulp fibers in a small amount to an extent that the thickness of the water-absorbent sheet composition would not increase.

The hydrophilic nonwoven fabric is preferably a nonwoven fabric having an appropriate basis weight and an appropriate thickness, from the viewpoint of giving the water-absorbent sheet composition of the present invention excellent liquid permeability, flexibility, strength and cushioning property, and speeding up the permeation rate of the water-absorbent sheet composition. The hydrophilic nonwoven fabric has a basis weight of preferably 25 g/m$^2$ or more, more preferably in the range of from 35 to 250 g/m$^2$, and even more preferably in the range of from 45 to 150 g/m$^2$. The hydrophilic nonwoven fabric has a thickness preferably in the range of from 200 to 1500 $\mu$m, more preferably in the range of from 250 to 1200 $\mu$m, and even more preferably in the range of from 300 to 1000 $\mu$m.

In the water-absorbent sheet composition of the present invention, it is more preferable that the sheet composition has a structure in which a primary absorbent layer and a secondary absorbent layer do not substantially mix with each other, from viewpoint of improving properties of a water-absorbent sheet composition having two absorbent layers and its stability in quality, and it is even more preferable that the sheet composition has a fractionated structure. The phrase "not substantially mix" as used herein means that a primary absorbent layer and a secondary absorbent layer do not inhibit each other in their respective properties, and the word "fractionated" means, in essence, immobilization of individual layers by sequential adhesion, or individual layer formation of a primary absorbent layer and a secondary absorbent layer by an insertion of a fractionating layer or the like. The structure in which the absorbent layers are not substantially mixed as mentioned above include, for example, a structure in which a primary absorbent layer and a secondary absorbent layer are individually laminated and bonded; a structure in which a primary absorbent layer and a secondary absorbent layer are separated by a breathable fractionating layer and bonded; and the like.

The above-mentioned breathable fractionating layer has appropriate breathability and liquid-permeability, and a particle-form substance such as a water-absorbent resin may be a layer that is substantially impermeable. Specific examples of the preferred materials therefor include at least one member selected from the group consisting of reticular products such as nets having fine pores made of PE or PP fibers; porous films such as perforated films; sanitary papers, such as tissue paper; cellulose-containing synthetic fiber nonwoven fabrics such as airlaid nonwoven fabrics made of pulp/PE/PP; rayon-containing synthetic fiber nonwoven fabrics such as spunlaid nonwoven fabrics made of rayon/PET. Among them, the rayon-containing synthetic fiber nonwoven fabrics are more preferably used, from the viewpoint of the properties of the resulting water-absorbent sheet composition. In these materials, the surface may be hydrophilically treated by a known method as occasion demands.

The thickness and the basis weight of the breathable fractionating layer are not particularly limited. Exemplifying more preferred forms, the breathable fractionating layer has a thickness preferably in the range of from 200 to 1200 $\mu$m, and more preferably in the range of from 250 to 800 $\mu$m. The breathable fractionating layer has a basis weight of preferably 10 g/m$^2$ or more, more preferably in the range of from 25 to 250 g/m$^2$, even more preferably in the range of from 40 to 150 g/m$^2$. The breathable fractionating layer preferably has a thickness of 1200 $\mu$m or less and a basis weight of 250 g/m$^2$ or less, from the viewpoint of thinning a water-absorbent sheet composition, and on the other hand, the breathable fractionating layer preferably has a thickness of 200 $\mu$m or more and a basis weight of 10 g/m$^2$ or more, from the viewpoint of obtaining sufficient strength against stretching and twisting during the production and upon use of a water-absorbent sheet composition.

The water-absorbent sheet composition of the present invention can be produced by a method, for example, as described in a method as described hereinbelow, utilizing a conventional method.

(a) A mixed powder of a water-absorbent resin and an adhesive is evenly dispersed over a hydrophilic nonwoven fabric, and passed through a heating furnace to fix the powder to an extent that the powder does not scatter, thereafter a mixed powder containing a water-absorbent resin different from that mentioned above is dispersed again, and a hydrophilic nonwoven fabric is overlaid thereto. The overlaid layers are together subjected to heat-and-pressure fusing.

(b) A mixed powder of a water-absorbent resin and an adhesive is evenly dispersed over a hydrophilic nonwoven fabric, a mixed powder containing a water-absorbent resin different from that mentioned above is dispersed again, and a hydrophilic nonwoven fabric is overlaid thereto. The overlaid layers are together subjected to heat-and-pressure fusing. (In this method, in order to avoid the mixing of each of the absorbent layers, it is preferable that a mixed powder containing a water-absorbent resin having a large specific gravity is dispersed, and a mixed powder containing a water-absorbent resin having a small specific gravity is then dispersed.)

(c) A mixed powder of a water-absorbent resin and an adhesive is evenly dispersed over a hydrophilic nonwoven fabric, a breathable fractionating layer is further overlaid thereto, a mixed powder containing a water-absorbent resin different from that mentioned above is further dispersed, and a hydrophilic nonwoven fabric is overlaid thereto. The overlaid layers are together subjected to heat-and-pressure fusing. (In this method, the overlaid products may be provided with pressure while heating even after a breathable fractionating layer is overlaid thereto.)

(d) An adhesive is melt-coated on a hydrophilic nonwoven fabric, immediately thereafter a water-absorbent resin is evenly dispersed to form a layer, and an adhesive is further melt-coated from an upper part to fix a water-absorbent resin. This intermediate product is again subjected to the same procedures as mentioned above using a water-absorbent resin different from that mentioned above.

A structure in which a primary absorbent layer and a secondary absorbent layer are not substantially mixed can be accomplished by, for example, producing a water-absorbent sheet composition according to the method shown in any one of these (a) to (d). Among them, the methods of (a), (c) and (d) are more preferred, from the viewpoint of being capable of fractionating a primary absorbent layer and a secondary absorbent layer, and the methods of (a) and (d) are even more preferred, from the viewpoint of convenience in the production method and high production efficiency. In the present specification, the above production method is also one of the present invention, and according to the above production method, fundamental properties as a water-absorbent sheet composition can be obtained at a high level, and a water-absorbent sheet composition which can accomplish thinning and avoidance of gel blocking phenomenon can be produced even while having a high content of a water-absorbent resin.

Here, among the methods exemplified in (a) to (d), the compositions can be produced by separately selecting methods of adhering a primary absorbent layer and a secondary absorbent layer, to be used in combination. The water-absorbent sheet composition may be subjected to emboss treatment during heat-and-pressure fusing in the production of a sheet or after the production of the sheet, for the purposes of improving the feel and improving strength of the water-absorbent sheet composition.

In addition, the water-absorbent sheet composition of the present invention may properly be formulated with an additive such as a deodorant, an anti-bacterial agent, or a gel stabilizer.

The water-absorbent sheet composition of the present invention has one feature in the viewpoint of enabling thinning of the composition. When the use in absorbent articles is taken into consideration, the water-absorbent sheet composition has a thickness, on a dry basis, of preferably 5 mm or less, more preferably 4 mm or less, even more preferably 3 mm or less, and still even more preferably from 1.0 to 2.5 mm.

Further, the water-absorbent sheet composition of the present invention has one feature in that a liquid has a fast permeation rate, and the water-absorbent sheet composition has a total permeation rate of preferably 120 seconds or less, more preferably 110 seconds or less, and even more preferably 100 seconds or less, when taking the use as an absorbent article into consideration.

Further, the water-absorbent sheet composition of the present invention has one feature in that a liquid has smaller liquid leakage, and the water-absorbent sheet composition has a leakage index of preferably 100 or less, more preferably 50 or less, and even more preferably 30 or less, when taking the use as an absorbent article into consideration.

A water-absorbent sheet composition satisfying all the properties as mentioned above is very highly preferable in consideration of its use as an absorbent article.

Further, since the water-absorbent sheet composition of the present invention has a very small amount of a material derived from nature, consideration has been made to the environment while having high performance in thickness, permeation rate, and a leakage index as mentioned above. The proportion of the natural material is preferably 25% or less, more preferably 15% or less, even more preferably 10% or less, and still even more preferably 5% or less. The proportion of the natural material is calculated by dividing a total content of pulp, cotton and the like contained in very small amounts as the constituents of the water-absorbent sheet composition by mass of the water-absorbent sheet composition.

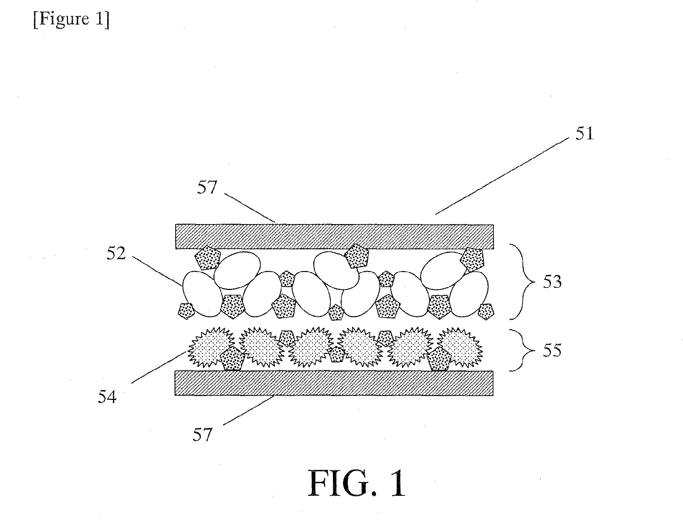

Next, the structure of the water-absorbent sheet composition of the present invention will be explained by referring to Figure 1. Here, Figure 1 is an enlarged cross-sectional view schematically showing one example of the structure of a water-absorbent sheet composition of the present invention.

A water-absorbent sheet composition 51 shown in Figure 1 comprises a primary absorbent layer 53 containing a first water-absorbent resin 52 and an adhesive, and a secondary absorbent layer 55 containing a second water-absorbent resin 54 and an adhesive. Here, the primary absorbent layer refers to a side to which a liquid to be absorbed is fed upon the preparation of an absorbent article using the water-absorbent sheet composition, and the secondary absorbent layer refers to a side to which a liquid to be absorbed via the primary absorbent layer is fed. Therefore, the water-absorbent sheet composition of the present invention has a structure in which a primary absorbent layer 53 and a secondary absorbent layer 55 are directly or indirectly (via a breathable fractionating layer or the like) overlaid in a thickness direction of a water-absorbent sheet composition 51, as shown in Figure 1.

Moreover, in Figure 1, a primary absorbent layer and a secondary absorbent layer are fractionated so that the two absorbent layers do not substantially mix with each other. A water-absorbent sheet composition 51 in Figure 1 has a four-layer structure, comprising a primary absorbent layer 53, a secondary absorbent layer 55, and front and back two layers made of a hydrophilic nonwoven fabric 57 positioned at each of the outer surface of the primary absorbent layer 53 and the secondary absorbent layer 55, which is a structure in which the absorbent layers are sandwiched by two or more sheets of hydrophilic nonwoven fabrics 57.

The absorbent article of the present invention has a structure in which a water-absorbent sheet composition of the present invention is sandwiched between a liquid-permeable sheet and a liquid-impermeable sheet. The absorbent article includes, for example, disposable diapers, incontinence pads, sanitary napkins, pet sheets, drip sheets for foods, water blocking materials for electric power cables, and the like. Further, as the liquid-permeable sheet and the liquid-impermeable sheet, known ones in the technical field of the absorbent articles can be used without particular limitations. The absorbent article can be produced by a known method.

EXAMPLES

The present invention will be specifically described hereinbelow by the Examples, without intending to limit the scope of the present invention thereto.

The properties of the water-absorbent resin and the water-absorbent sheet composition were measured in accordance with the following methods.

< Water-Retention Capacity of Saline Solution of Water-Absorbent Resin >

The amount 2.0 g of water-absorbent resin was weighed in a cotton bag (Cottonbroad No. 60, width 100 mm × length 200 mm), and placed in a 500 mL-beaker. Physiological saline (0.9% by mass aqueous solution of sodium chloride, hereinafter referred to the same) was poured into the cotton bag in an amount of 500 g at one time, and the physiological saline was dispersed so as not to cause an unswollen lump of the water-absorbent resin. The upper part of the cotton bag was tied up with a rubber band, and the cotton bag was allowed to stand for 1 hour, to sufficiently make the water-absorbent resin swell. The cotton bag was dehydrated for 1 minute with a dehydrator (manufactured by Kokusan Enshinki Co., Ltd., product number: H-122) set to have a centrifugal force of 167G. The mass Wa (g) of the cotton bag containing swollen gels after the dehydration was measured. The same procedures were carried out without adding water-absorbent resin, and the empty mass Wb (g) of the cotton bag upon wetting was measured. The water-retention capacity of saline solution of the water-absorbent resin was calculated from the following formula.

$$\text{Water-Retention Capacity of Saline Solution (g/g) of}$$
$$\text{Water-Absorbent Resin}$$
$$= [\text{Wa} - \text{Wb}] \ (\text{g})/\text{Mass} \ (\text{g}) \ \text{of Water-Absorbent Resin}$$

< Initial Water Absorption Rate and Effective Amount of Water Absorbed of Water-Absorbent Resin >

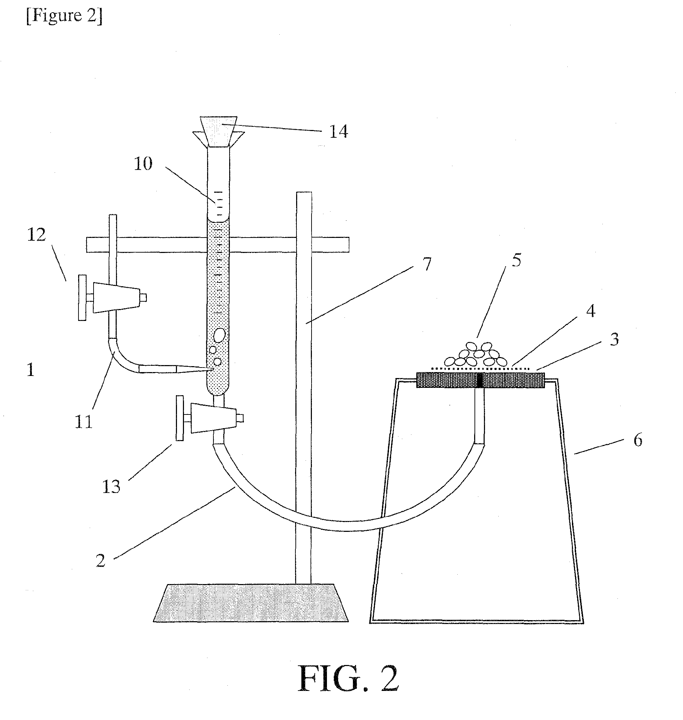

The initial water absorption rate and the effective amount of water absorbed of the water-absorbent resin were measured using a measurement apparatus as shown in Figure 2.

The measurement apparatus comprised a burette section 1, a lead tube 2, a measuring platform 3, a nonwoven fabric 4, a stand 6, and a clamp 7. The burette section 1 was connected to a rubber plug 14 at the top of a burette 10, and an air inlet tube 11 and a cock 12 at the bottom portion thereof, and further, the burette 10 had a cock 13 at a tip end of bottom portion thereof. The burette section 1 was fixed with a clamp 7. The lead tube 2 was attached between the burette section 1 and the measuring platform 3. The lead tube 2 had an inner diameter of 6 mm. A hole of a diameter of 2 mm is made at the central section of the measuring platform 3, and the lead tube 2 is connected thereto. The measuring platform 3 was supported at an appropriate height by a stand 6.

The measurements of the initial water absorption rate and the effective amount of water absorbed using the measurement apparatus as described above were carried out by the following procedures. The measurements were taken indoors at a temperature of 25°C and humidity of from 45 to 75%. First, the cock 12 and the cock 13 at the burette section 1 were closed, and a 0.9% by mass aqueous sodium chloride solution adjusted to 25°C was poured from the top of the burette 10 and the top of the burette was plugged with the rubber plug 14. Thereafter, the cock 12 and the cock 13 at the burette section 1 were opened. Next, an internal of a lead tube 2 was filled with a 0.9% by mass aqueous sodium chloride solution while removing bubbles, and the height of the measuring platform 3 was adjusted so that a water level of a 0.9% by mass aqueous sodium chloride solution coming out of a lead tube inlet at the central portion of the measuring platform 3 and an upper side of the measuring platform 3 would be at the same height.

Next, a nonwoven fabric 4 cut into dimensions of 30 mm × 30 mm (hydrophilic rayon spun laid having a basis weight of 25 g/m²) was spread on a lead tube inlet at the central portion of the measuring platform 3, and the nonwoven fabric was allowed to absorb water until reaching an equilibrium. In the state where a nonwoven fabric absorbed water, the generation of bubbles from an air lead tube 11 to a burette 10 was observed, and having confirmed that the generation of bubbles stopped within several minutes, it was judged that an equilibrium was reached. After equilibration, the scales of the burette 10 were read off to confirm a zero point.

Separately, 0.10 g of a water-absorbeut resin 5 was measured accurately, and supplied at one time to a central part of a nonwoven fabric 4. An amount of a 0.9% by mass aqueous sodium chloride solution reduced inside the burette 10 (in other words, an amount of a 0.9% by mass aqueous sodium chloride solution absorbed by the particles of a water-absorbent resin 5) was sequentially read off, and a reduced portion of a 0.9% by mass aqueous sodium chloride solution after 30 seconds counted from the supplying of a water-absorbent resin 5 Wc (mL) was recorded as an amount of water absorbed per 0.10 g of a water-absorbent resin. Incidentally, the measurement of the reduced portion was continued to be measured even after the passage of 30 s, and the measurement was completed after 30 minutes, The measurements were taken 5 times per one kind of a water-absorbent resin, and a 3-point average excluding a minimum value and a maximum value was used.

The amount of a 0.9% by mass aqueous sodium chloride solution absorbed to a water-absorbent resin 5 after 30 s from the supply Wc (mL) was converted to an amount of water absorbed per 1 g of a water-absorbent resin, and a quotient obtained by further dividing the resulting converted value by 30 (s) was defined as an initial water absorption rate (mL/s) of the water-absorbent resin. In other words, the initial water absorption rate (mL/s) $= Wc \div (0.10 \times 30)$.

In addition, an amount of water absorbed after the passage of 30 minutes from the supply of a water-absorbent resin 5 Wd (mL) was converted to an amount of water absorbed per 1 g of a water-absorbent resin, and defined as an effective amount of water absorbed (mL/g) of saline solution of the water-absorbent resin. In other words, the effective amount of water absorbed (mL/g) $= Wd \div 0.10$.

< Water Absorption Rate of Saline Solution of Water-Absorbent Resin >

This test was conducted in a room temperature-controlled to 25˚ $\pm$ 1˚C. The amount 50 $\pm$ 0.1 g of physiological saline was weighed out in a 100 mL beaker, and a magnetic stirrer bar (8 mm$\phi$ $\times$ 30 mm, without a ring) was placed therein. The beaker was immersed in a thermostat, of which liquid temperature was controlled to 25˚ $\pm$ 0.2˚C. Next, the beaker was placed over the magnetic stirrer so that a vortex was generated in physiological saline at a rotational speed of 600 r/min, the water-absorbent resin was then quickly added in an amount of 2.0 $\pm$ 0.002 g to the above beaker, and the time period (seconds) from a point of addition of the water-absorbent resin to a point of convergence of the vortex of the liquid surface was measured with a stopwatch , which was defined as a water absorption rate of the water-absorbent resin.

< Median Particle Size of Water-Absorbent Resin >

Unless specified otherwise, the particle size of the water-absorbent resin is defined as a median particle size, and measured as follows. An amorphous silica (Sipernat 200, Degussa Japan) was mixed in an amount of 0.5 g as a lubricant with 100 g of a water-absorbent resin.

The above-mentioned water-absorbent resin particles were allowed to pass though a JIS standard sieve having a sieve opening of 250 $\mu$m, and a median particle size was measured using a combination of sieves of (A) in a case where the particles are allowed to pass in an amount of 50% by mass or more, or a combination of sieves of (B) in a case where 50%a by mass or more of the particles remain on the sieve.

(A) JIS standard sieves, a sieve having an opening of 425 $\mu$m, a sieve having an opening of 250 $\mu$m, a sieve having an opening of 180 $\mu$m, a sieve having an opening of 150 $\mu$m, a sieve having an opening of 106 $\mu$m, a sieve having an opening of 75 $\mu$m, a sieve having an opening of 45 $\mu$m, and a receiving tray were combined in order from the top.

(B) JIS standard sieves, a sieve having an opening of 850 $\mu$m, a sieve having an opening of 600 $\mu$m, a sieve having an opening of 500 $\mu$m, a sieve having an opening of 425 $\mu$m, a sieve having an opening of 300 $\mu$m, a sieve having an opening of 250 $\mu$m, a sieve having an opening of 150 $\mu$m, and a receiving tray were combined in order from the top.

The above-mentioned water-absorbent resin particles were placed on an uppermost sieve of the combined sieves, and shaken for 20 minutes with a rotating and tapping shaker machine to classify the particles.

After classification, the relationships between the opening of the sieve and an integral of a mass percentage of the water-absorbent resin remaining on the sieve were plotted on a logarithmic probability paper by calculating the mass of the water-absorbent resin remaining on each sieve as a mass percentage to an entire amount, and accumulating the mass percentages in order, starting from those having larger particle diameters. A particle diameter corresponding to a 50% by mass cumulative mass percentage was regarded as a median particle size by joining the plots on the probability paper in a straight line.

< Bulk Specific Gravity of Water-Absorbent Resin >

The bulk specific gravity was measured as prescribed in "4.3 Bulk Specific Gravity" of a vinyl chloride resin test method, JIS K6720-2 (1999).

< Measurement of Thickness of Water-Absorbent Sheet Composition >

The thickness of the resulting water-absorbent sheet composition was measured using a thickness measurement instrument (manufactured by Kabushiki Kaisha Ozaki Seisakusho, model number: J-B). As the measurement sites, three sites were arbitrarily determined in a lengthwise direction, on the left end, the center, and the right end; for example, in a water-absorbent sheet composition of 10 cm × 30 cm, the left end was set at a site 3 cm away from the left side, the center was set at a site 15 cm away therefrom, and the right end was set at a site 27 cm away therefrom. As the width direction, a uniform central part was measured. The measurement value for thickness was obtained by measuring three times at each site and averaging the values for each site. Further, the values at the left end, the center, and the right end were averaged, to give a thickness of an overall water-absorbent sheet composition.

< Strength of Water-Absorbent Sheet Composition >

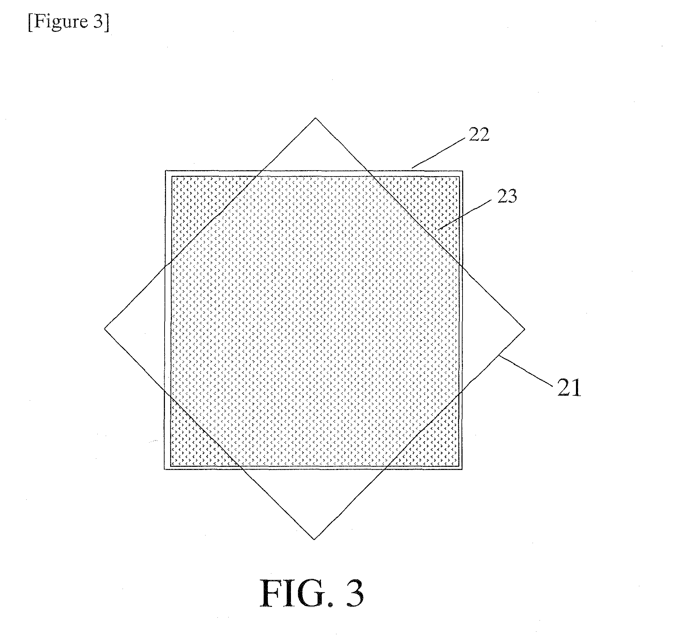

The strength of the water-absorbent sheet composition was evaluated in accordance with the following method. The resulting water-absorbent sheet composition was cut into a size of 10 cm × 10 cm. Next, the entire side of each of one side of two pieces of acrylic plates of 10 cm × 10 cm (mass about 60g) was adhered with a double-sided adhesive tape. As shown in Figure 3, the acrylic plates were pressed in a manner that the diagonal lines of acrylic plates 21, 22 form 45 degrees in angle, sandwiching from top and bottom to fix so that the double-sided adhesive tape faces the side of the water-absorbent sheet composition 23.

The strength-test pieces of the water-absorbent sheet composition prepared in the manner as described above were placed on a metallic tray of sieves, used in the section of the above-mentioned < Median Particle Size of Water-Absorbent Resin >, and a lid was put thereon. Thereafter, the lidded vessel was tapped with rotations with a rotating and tapping shaker machine for 3 minutes (at this time, a few layers of mesh sieves may be provided as a spacer between the tray and the tapping machine). The strength of the water-absorbent sheet composition was evaluated based on the external appearance after tapping in accordance with the following criteria.

○: The water-absorbent sheet composition showed no changes in external appearance, and did not easily move even when the acrylic plates were tried to be displaced.

△: The water-absorbent sheet composition showed no changes in external appearance, but the water-absorbent sheet composition was removed from the center when the acrylic plates were displaced.

×: The water-absorbent sheet composition was split in two from the center, and the contents were scattered.

< Feel of Water-Absorbent Sheet Composition >

The feel of a water-absorbent sheet composition was evaluated by the following method. A water-absorbent sheet composition obtained was cut into a size of 10 cm × 10 cm. Ten panelists were selected, and the panelists were asked to make a three-rank evaluation for feel in accordance with the following criteria, evaluation scores of the panelists were averaged to evaluate feel of a water-absorbent sheet composition.

Rank A: The feel of the surface is smooth and soft, giving a pleasant feel, and there is no deposition (evaluation score: 5).

Rank B: The feel of the surface is smooth but feels granular texture. A very small amount of deposition is found on the surface (evaluation score: 3).

Rank C: The surface has a rugged feel, making its feel unpleasant. Deposition of powder is found on the surface (evaluation score: 1).

< Operability During the Production of Water-Absorbent Sheet Composition >

The same water-absorbent sheet composition was produced continuously 5 times, in accordance with the production process described in Examples and Comparative Examples described later.

Three operating individuals were asked to make a 3-rank evaluation on the state of the water-absorbent sheet composition and a sheet production machine, as regarding a water-absorbent resin powder after the production, and the evaluation scores for each item were averaged, and operability during the production of a water-absorbent sheet composition was evaluated.

[Table 1]

Table 1

| Evaluation Items | Evaluation Criteria | Evaluation Score |
|---|---|---|
| Spreading State of Mixture | The spreading state is uniform and has high reproducibility. | 5 |
| | Variance and deviation are found in the spreading state. | 3 |
| | In the spreading state variance and deviation are generated in many cases. | 1 |
| Powder Deposition to Spreader Roller | Deposition to a roller is hardly found. | 5 |
| | Deposition is found in a small amount but removable with air-wetting | 3 |
| | Amount of deposition is in a large amount, thereby making it necessary to remove with a hard brush. | 1 |
| Powder Scattering in the Surroundings of Spreader | There is hardly any powder scattering to those other than nonwoven fabric. | 5 |
| | Powder is scattered in a wide range over a conveyor. | 3 |
| | Powder is scattered even on floor of room. | 1 |

< Evaluations of Total Permeation Rate and Amount of Re-wet of Water-Absorbent Sheet Composition >

A rectangular strip of a water-absorbent sheet composition of 10 × 30 cm, cut in a manner that a longitudinal direction thereof is to be in a length direction (machine feeding direction) of the hydrophilic nonwoven fabric, was used as a sample.

In a 10 L vessel were placed 60 g of sodium chloride, 1.8 g of calcium chloride dihydrate, 3.6 g of magnesium chloride hexahydrate, and a proper amount of distilled water to completely dissolve. Next, 15 g of an aqueous 1% by mass poly(oxyethylene) isooctylphenyl ether solution was added thereto, and distilled water was further added to adjust the weight of the overall aqueous solution to 6000 g. Thereafter, the mixed solution was colored with a small amount of Blue No. 1 to prepare a test solution.

On an upper part of a sample (water-absorbent sheet composition) was placed a polyethylene air-through style porous liquid-permeable sheet having the same size as the sample (10 × 30 cm) and a basis weight of 22 g/m$^2$. In addition, underneath the sample was placed a polyethylene liquid-impermeable sheet having the same size and basis weight as the sheet, to prepare a simple body liquid-absorbent article. A cylindrical cylinder having an inner diameter of 3 cm was placed near the central section of this body liquid-absorbent article, and a 50 mL test solution was supplied thereto at one time. At the same time, a time period until the test solution was completely permeated into the body liquid-absorbent article was measured with a stopwatch, which was referred to as a first permeation rate (sec). Next, the same procedures were carried out 30 minutes thereafter and 60 minutes thereafter, to measure second and third permeation rates (see). A total of the number of seconds for the first to third permeation rates was referred to as a total permeation rate.

After 120 minutes from the start of the feed of the first test liquid, the cylinder was removed, filter papers of 10 cm each side, of which mass was previously measured (We (g), about 70 g), were stacked near the liquid feeding position of the body liquid-absorbent article, and a 5 kg weight having a size of 10 cm × 10 cm was placed thereon. After 5 minutes of applying a load, the mass (Wf (g)) of the filter papers was measured, and an increased mass was defined as the amount of re-wet (g) as follows.

$$\text{Amount of Re-wet (g)} = \text{Wf} - \text{We}$$

< Slope Leakage Test >

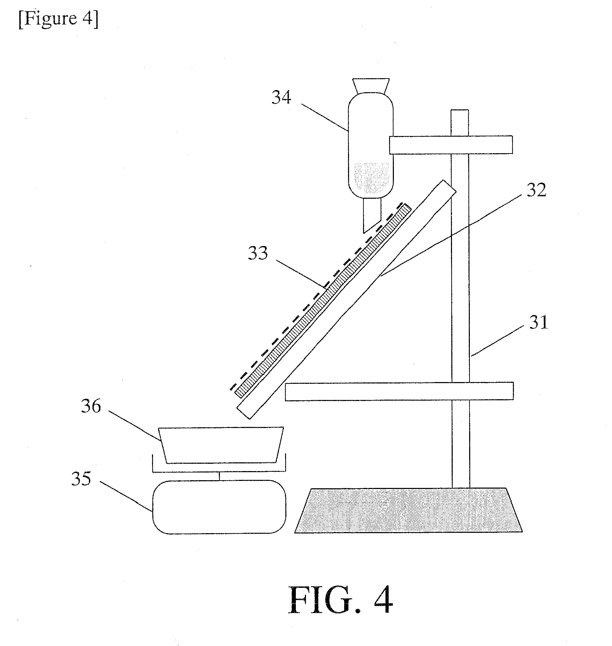

A slope leakage test was conducted using an apparatus shown in Figure 4. Schematically, a mechanism is as follows. A commercially available stand 31 for experimental facilities was used to slope an acrylic plate 32 and fixed, the above-mentioned test solution was then supplied to a water-absorbent sheet composition 33 placed on the acrylic plate from a dropping funnel 34 positioned vertically above the sheet composition, and a leakage amount was measured with a balance 35. The detailed specifications are given hereinbelow.

An acrylic plate 32 has a length in the direction of the slope plane of 45 cm, and fixed so that an angle formed with a stand 31 against the horizontal is $45° \pm 2°$. The acrylic plate 32 had a width of about 100 cm and a thickness of about 1 cm, and plural water-absorbent sheet compositions 33 could be concurrently measured. The acrylic plate 32 had a smooth surface, so that a liquid was not detained or absorbed to the plate.

A dropping funnel 34 was fixed at a position vertically above the sloped acrylic plate 32 using a stand 31. The dropping funnel 34 had a volume of 100 mL, and an inner diameter of a tip end portion of about 4 mm$\phi$, and an aperture of the cock was adjusted so that a liquid was supplied at a rate of 8 mL/seconds.

A balance 35 on which a metallic tray 36 was placed was set at a lower part of the acrylic plate 32, and all the test solutions flowing down the acrylic plate was received as leakage, and its mass was recorded to the accuracy of 0.1 g.

A slope leakage test using an apparatus as described above was carried out in accordance with the following procedures. The mass of a water-absorbent sheet composition 33 cut into a size of a length of 30 cm and a width of 10 cm was measured, and an air through-style polyethylene liquid-permeable nonwoven fabric (basis weight: 22 g/m$^2$) of the same size was attached from an upper part thereof, and further a polyethylene liquid-impermeable sheet having the same basis weight of the same size was attached from a lower part thereof to prepare a simple absorbent article. The simple absorbent article was adhered on the acrylic plate 32 (in order not to stop leakage intentionally, the bottom end of the water-absorbent sheet composition 33 was not adhered to the acrylic plate 32).

Marking was put on the water-absorbent sheet composition 33 at a position 2 cm away in a downward direction from a top end thereof, and a supplying inlet for the dropping funnel 34 was fixed so that the inlet was positioned at a distance 8 mm $\pm$ 2 mm vertically above the marking.

A balance 35 was turned on, and the indication was tared so that the indication was zero, and thereafter 80 mL of the above-mentioned test solution was supplied at one time to the dropping funnel 34. An amount of liquid poured into a metallic tray 36 after the test solution was allowed to flow over a sloped acrylic plate 32 without being absorbed into a water-absorbent sheet composition 33 was measured, and this amount of liquid is referred to a first leakage amount (mL). The numerical value for this first leakage amount (mL) was denoted as LW1.

Second and third test solutions were supplied in 10-minute intervals from the beginning of the first supply, and second and third leakage amounts (mL) were measured, and the numerical values therefor were respectively denoted as LW2 and LW3.

Next, a leakage index was calculated in accordance with the following equation. The more the index approaches to zero, the smaller the leakage amount at a slope of a water-absorbent sheet composition, especially an initial leakage amount, whereby it is judged to be an excellent water-absorbent sheet composition.

$$\text{Leakage Index:} \quad L = LW1 \times 10 + LW2 \times 5 + LW3$$

(Production Example 1: Production of Water-Absorbent Resin A)

A cylindrical round bottomed separable flask having an internal diameter of 100 mem, equipped with a reflux condenser, a dropping funnel, a nitrogen gas inlet tube, and a stirrer having two steps of a stirring blade having 4 inclined paddle blades with a blade diameter of 50 mm was furnished. This flask was charged with 500 mL of n-heptane, and 0.92 g of a sucrose stearate having an HLB of 3 (manufactured by Mitsubishi-Kagaku Foods Corporation, Ryoto sugar ester S-370) and 0.92 g of a maleic anhydride-modified ethylene-propylene copolymer (manufactured by Mitsui Chemicals, Inc., Hi-wax 1105A) were added thereto as surfactants. The temperature was raised to 80°C to dissolve the surfactants, and thereafter the solution was cooled to 50°C.

On the other hand, a 500 mL-Erlenmeyer flask was charged with 92 g of an 80.5% by mass aqueous solution of acrylic acid, and 154.1 g of a 20.0% by mass aqueous sodium hydroxide was added dropwise thereto with cooling from external to neutralize 75% by mol. Thereafter, 0.11 g of potassium persulfate and 9.2 mg of N,N'-methylenebisacrylamitle were added thereto to dissolve, to prepare an aqueous monomer solution for the first step.

The above-mentioned aqueous monomer solution was added to the above-mentioned separable flask, while setting a rotational speed of a stirrer to 450 rpm, and the temperature was kept at 35 °C for 30 minutes, while replacing the internal of the system with nitrogen. Thereafter, the flask was immersed in a water bath kept at 70°C, and a polymerization was carried out, to give a slurry after the first-step polymerization.

On the other hand, another 500 mL-Erlenmeyer flask was charged with 128.8 g of an 80.5% by mass aqueous solution of acrylic acid, and 174.9 g of a 24.7% by mass aqueous sodium hydroxide was added dropwise thereto with cooling from external to neutralize 75% by mol. Thereafter, 0.16 g of potassium persulfate and 12.9 mg of N,N'-methylenebisacrylamide added thereto to dissolve, to prepare an aqueous monomer solution for the second step.

The agitation rotational speed of the stirrer containing the slurry after the polymerization mentioned above was changed to 1000 rpm, and the temperature was then cooled to 25˚C. The aqueous monomer solution for the second step mentioned above was added to the internal of the system, and the temperature was held for 30 minutes while replacing nitrogen. The flask was again immersed in a water bath at 70˚C, and the temperature was raised to carry out polymerization, to give a slurry after the second-step polymerization.

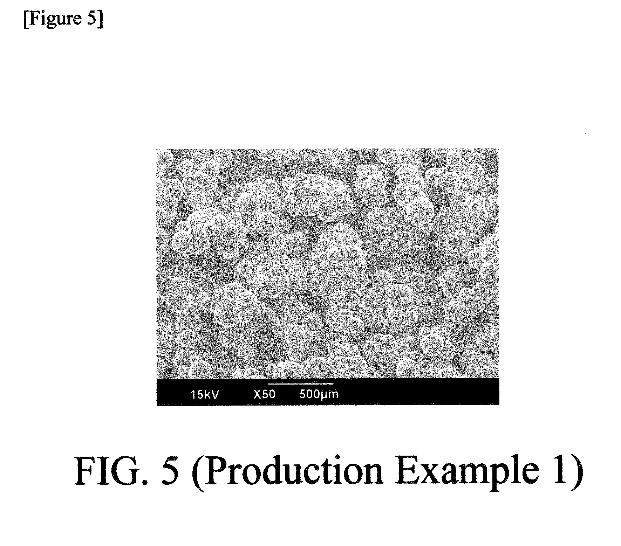

Next, the temperature was raised using an oil bath at 120˚C, and water and n-heptane were subjected to azeotropic distillation to remove 275,3 g of water to the external of the system, while refluxing n-heptane. Thereafter, 8.83 g of a 2% aqueous solution of ethylene glycol diglycidyl ether was added thereto, and the mixture was kept at 80˚C for 2 hours. Subsequently, n-heptane was evaporated to dryness, to give 231.2 g of a water-absorbent resin A in the form in which spherical particles are agglomerated as shown in Figure 5. The resulting water-absorbent resin A had a median particle size of 340 $\mu$m, a water-retention capacity of saline solution of 34 g/g, and a bulk specific gravity of 0.7 g/mL. Other properties are as shown in Table 2.

(Production Example 2: Production of Water-Absorbent Resin B)

The same procedures as in Production Example of Water-Absorbent Resin A were carried out except that the rotational speed of a stirrer during a first-step polymerization was changed to 400 rpm, and that the amount of a 2% aqueous solution of ethylene glycol diglycidyl ether added after the removal of water to the external of the system by azeotropic distillation was changed to 6.62 g, in Production Example of Water-Absorbent Resin A, to give 232.0 of a water-absorbent resin B in the form in which the spherical particles were agglomerated. The resulting water-absorbent resin B had a median particle size of 390 $\mu$m, a water-retention capacity of saline solution of 42 g/g, and a bulk specific gravity of 0.7 g/mL. Other properties are as shown in Table 2.

(Production Example 3: Production of Water-Absorbent Resin C)

A cylindrical round bottomed separable flask having an internal diameter of 100 mm, equipped with a reflux condenser, a dropping funnel, a nitrogen gas inlet tube, and a stirrer having two steps of a stirring blade having 4 inclined paddle blades with a blade diameter of 50 mm was furnished. This flask was charged with 550 mL of n-heptane, and 0.84 g of sorbitan monolaurate having an HLB of 8.6 (manufactured by NOF Corporation, Nonion LP-20R) was added thereto as a surfactant. The temperature was raised to 50˚C to dissolve the surfactants, and thereafter the solution was cooled to 40˚C.

On the other hand, a 500 mL-Erlenmeyer flask was charged with 70 g of an 80.5% by mass aqueous solution of acrylic acid, and 112.3 g of a 20.9% by mass aqueous sodium hydroxide was added dropwise thereto while ice-cooling to neutralize 75% by mol. Thereafter, 0.084 g of potassium persulfate was added thereto to dissolve, to prepare an aqueous monomer solution.

The above-mentioned aqueous monomer solution was added to the above-mentioned separable flask, while setting a rotational speed of a stirrer to 800 rpm. The internal of the system was replaced with nitrogen for 30 minutes, and the flask was immersed in a water bath kept at 70˚C to raise the temperature, and a polymerization reaction was carried out for 2 hours.

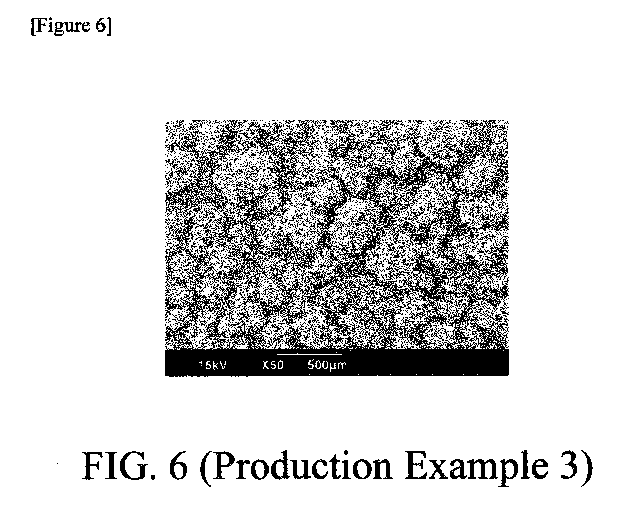

Next, the temperature was raised using an oil bath at 120˚C, and water and n-heptane were subjected to azeotropic distillation to remove 85.5 g of water to the external of the system, while refluxing n-heptane. Thereafter, 3.50 g of a 2% aqueous solution of ethylene glycol diglycidyl ether was added thereto, and the mixture was kept at 80˚C for 2 hours. Subsequently, n-heptane was evaporated to dryness, to give 72.3 g of a watcr-absorbent resin C in a granular form as shown in Figure 6. The resulting water-absorbent resin C had a median particle size of 240 $\mu$m, a water-retention capacity of saline solution of 38 g/g, a bulk specific gravity of 0.4 g/mL, and an effective amount of water absorbed of 63 mL/g.

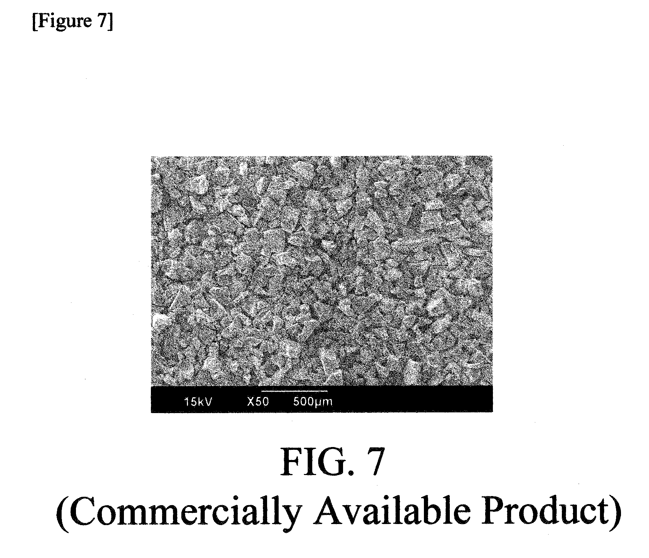

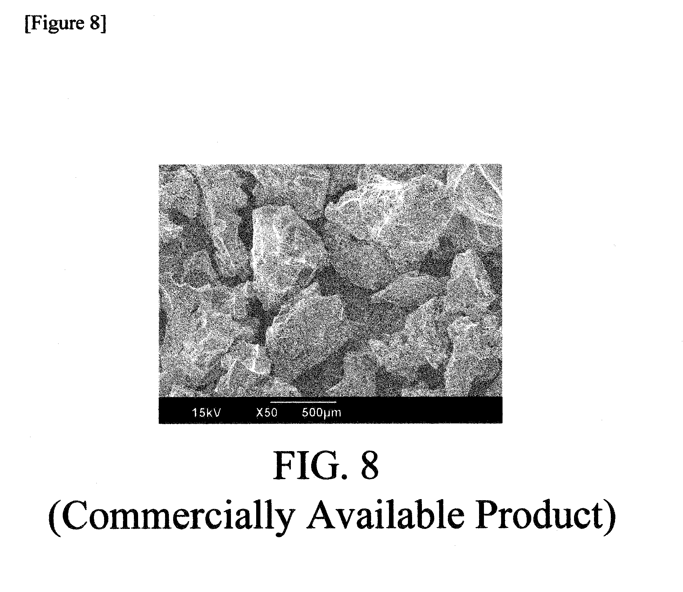

Commercially available water-absorbent resins D and E, aqueous solution polymerization products, were used in experiments. A water-absorbent resin D is a fine powder having a irregular and pulverized shape as shown in Figure 7, and the water-absorbent resin had a median particle size of 90 $\mu$m, a water-retention capacity of saline solution of 29 g/g, a bulk specific gravity of 0.7 g/mL, and an effective amount of water absorbed of 39 mL/g. On the other hand, a water-absorbent resin E is a coarse powder having a irregular and pulverized shape as shown in Figure 8, and the water-absorbent resin had a median particle size of 610 $\mu$m, a water-retention capacity of saline solution of 32 g/g, and a bulk specific gravity of 0.6 g/mL. Other properties are as shown in Table 2.

(Example 1)

A roller spreader (manufactured by HASf-ITMA CO., LTD., SINTERACE M/C) was charged at its supplying inlet with a mixture prepared by homogeneously mixing 55 parts by mass of an ethylene-vinyl acetate copolymer (melting temperature: 95˚C) as an adhesive and 270 parts by mass of a water-absorbent resin A (median particle size: 340 $\mu$m; water-absorption rate of saline solution: 38 s, initial water-absorption rate: 0.17 mL/s, effective amount of water absorbed: 56 mL/g). On the other hand, a hydrophilic nonwoven fabric made of rayon having a width of 30 cm (basis weight: 45 g/m², rayon content: 100%) was spread over a conveyor at the bottom part of the spreader. Next, the spreading roller and the bottom part conveyor were operated, thereby allowing the above-mentioned mixture to evenly overlay the above-mentioned nonwoven fabric at a basis weight of 325 g/m². The resulting overlaid product on a conveyor was allowed to pass through a heating furnace (set temperature: 110˚C) also provided in the above-mentioned roller spreader, and then cooled to room temperature, to give an intermediate product of a water-absorbent sheet composition in which a primary absorbent layer was formed.

Separately, a roller spreader (manufactured by HASHIMA CO., LTD., SINTERACE M/C) was charged at its supplying inlet with a mixture prepared by homogeneously mixing 55 parts by mass of an ethylene-vinyl acetate copolymer (melting temperature: 95˚C) as an adhesive and 270 parts by mass of a water-absorbent resin C (median particle size: 240 $\mu$m; water-absorption rate of saline solution: 3 s). An intermediate product of a water-absorbent sheet composition mentioned above was spread over a conveyor of the roller spreader, and the spreading roller and the bottom part conveyor were operated, thereby allowing the above-mentioned mixture to evenly overlay over the above-mentioned intermediate product at a basis weight of 78 g/m².

The overlaid product obtained was pressed from a top part with the above-mentioned hydrophilic nonwoven fabric made of rayon, and heat-fused with a thermal laminating machine (manufactured by HASHIMA CO., LTD., straight linear fusing press HP-600LF) set at 130C to integrate, to give a water-absorbent sheet composition. The cross section of the structure of the resulting water-absorbent sheet composition, as schematically shown, had a structure as shown in Figure 1.

The water-absorbent sheet composition was cut into a given size to measure the properties of the water-absorbent sheet composition. The results are shown in Table 3.

(Example 2)

A water-absorbent sheet composition was obtained in the same manner as in Example 1, except that the basis weight of a mixture of a water-absorbent resin C and an ethylene-vinyl acetate copolymer spread in the second time in Example 1 was changed to 36 g/m². The resulting water-absorbent sheet composition was cut into a given size to measure the properties of the water-absorbent sheet. The results are shown in Table 3.

(Example 3)

A water-absorbent sheet composition was obtained in the same manner as in Example 1, except that in Example 1 a water-absorbent resin A spread in the first time was changed to a water-absorbent resin B (median particle size: 390 $\mu$m; water-absorption rate of saline solution: 53 s; initial water-absorption rate: 0.23 mL/s; an effective amount of water absorbed: 63 mL/g), that an adhesive an ethylene-vinyl acetate copolymer was changed to a copolymer polyester (melting point: 80˚C), that a hydrophilic nonwoven fabric made of rayon was changed to a hydrophilic nonwoven fabric made of rayon-PET (basis weight: Sdg/m², rayon content: 70%), and that a heating temperature of a thermal laminating machine was changed to 100˚C. The resulting water-absorbent sheet composition was cut into a given size to measure the properties of the water-absorbent sheet. The results are shown in Table 3.

(Example 4)

A water-absorbent sheet composition was obtained in the same manner as in Example 3, except that the basis weight of a mixture of a copolymer polyester spread in the second time in Example 3 (melting temperature: 80˚C) and a water-absorbent resin C was changed to 36 g/m². The resulting water-absorbent sheet composition was cut into a given size to measure the properties of the water-absorbent sheet. The results are shown in Table 3.

(Example 5)

A roller spreader (manufactured by HASHIMA CO., LTD., SINTERACE M/C) was charged at its supplying inlet with a mixture prepared by homogeneously mixing 100 parts by mass of a low-density polyethylene (melting temperature: 107˚C) as an adhesive and 400 parts by mass of a water-absorbent resin A (median particle size: 340 $\mu$m; water-

absorption rate of saline solution: 38 s, initial water-absorption rate: 0.17 mL/s, effective amount of water absorbed: 56 mL/g). On the other hand, a hydrophilic nonwoven fabric made of rayon-PET having a width of 30 cm (basis weight: 50 g/m$^2$, rayon content: 70%) was spread over a conveyor at the bottom part of the spreader. Next, the spreading roller and the bottom part conveyor were operated, thereby allowing the above-mentioned mixture to evenly overlay over the above-mentioned nonwoven fabric at a basis weight of 500 g/m$^2$.

The overlaid product obtained was pressed from a top part with the hydrophilic nonwoven fabric made of rayon-PET (basis weight: 40 g/m$^2$, rayon content: 60%) as a breathable fractionating layer, and heat-fused with a thermal laminating machine (manufactured by HASHIMA CO., LTD., straight linear fusing press HP-600LF) set at 140˚C to integrate, to give an intermediate product of a water-absorbent sheet composition.

Separately, a roller spreader (manufactured by HASHIMA CO., LTD., SINTERACE M/C) was charged at its supplying inlet with a mixture prepared by homogeneously mixing 16 parts by mass of a low-density polyethylene (melting temperature: 107˚C) as an adhesive and 65 parts by mass of a water-absorbent resin C (median particle size: 240 $\mu$m; water-absorption rate of saline solution: 3 s). An intermediate product of a water-absorbent sheet composition mentioned above was spread over a conveyor of the roller spreader so that the breathable fractionating layer was located on an upper side, and the spreading roller and the bottom part conveyor were operated, thereby allowing the above-mentioned mixture to evenly overlay over the above-mentioned intermediate product at a basis weight of 81 g/m$^2$.

The overlaid product obtained was pressed from a top part with the above-mentioned hydrophilic nonwoven fabric made of rayon-PET, and heat-fused with a thermal laminating machine (manufactured by HASHIMA CO., LTD., straight linear fusing press HP-600LF) set at 140˚C to integrate, to give a water-absorbent sheet composition. The water-absorbent sheet composition was cut into a given size to measure the properties of the water-absorbent sheet composition. The results are shown in Table 3.

(Example 6)

A water-absorbent sheet composition was obtained in the same manner as in Example 5, except that, in Example 5, the amounts of a water-absorbent resin A, a water-absorbent resin C, and an adhesive used were changed to those as listed in Table 2, and that a hydrophilic nonwoven fabric used was changed to be the same one as a nonwoven fabric used as a breathable fractionating layer, in other words, a hydrophilic nonwoven fabric made of rayon-PET (basis weight: 40 g/m$^2$, rayon content: 60%). The resulting water-absorbent sheet composition was cut into a given size to measure the properties of the water-absorbent sheet. The results are shown in Table 3.

(Comparative Example 1)

A roller spreader (manufactured by HASHIMA CO., LTD., SINTERACE M/C) was charged at its supplying inlet with a mixture prepared by homogeneously mixing 55 parts by mass of the above-mentioned ethylene-vinyl acetate copolymer as an adhesive and 340 parts by mass of the above-mentioned water-absorbent resin A. On the other hand, a hydrophilic nonwoven fabric made of rayon having a width of 30 cm (basis weight: 45 g/m$^2$, rayon content: 100%) was spread over a conveyor at the bottom part of the spreader. Next, the spreading roller and the bottom part conveyor were operated, thereby allowing the above-mentioned mixture to evenly overlay over the above-mentioned nonwoven fabric at a basis weight of 395 g/m$^2$.

The overlaid product obtained was pressed from a top part with the above-mentioned hydrophilic nonwoven fabric made of rayon, and heat-fused with a thermal laminating machine (manufactured by HASHIMA CO., LTD., straight linear fusing press HP-600LF) set at 130˚C to integrate, to give a water-absorbent sheet composition. The water-absorbent sheet composition was cut into a given size to measure the properties of the water-absorbent sheet composition. The results are shown in Table 3.

(Comparative Example 2)

A water-absorbent sheet composition was obtained in the same manner as in Comparative Example 1, except that the spreader was charged at a supplying inlet a mixture prepared by homogeneously mixing 68 parts by mass of the above-mentioned ethylene-vinyl acetate copolymer as an adhesive, and 270 parts by mass of the water-absorbent resin A, and 65 parts by mass of the water-absorbent resin C, and the above-mentioned mixture was spread at one time at a basis weight of 403 g/m$^2$, in Comparative Example 1. The resulting water-absorbent sheet composition was cut into a given size to measure the properties of the water-absorbent sheet. The results are shown in Table 3.

(Comparative Example 3)

A water-absorbent sheet composition was obtained in the same manner as in Example 3, except that a water-absorbent rein C in Example 3 was changed to a water-absorbent resin D having a irregular and pulverized shape (median particle size: 90 μm; water-absorption rate of saline solution: 7 s). The resulting water-absorbent sheet composition was cut into a given size to measure the properties of the water-absorbent sheet. The results are shown in Table 3.

(Comparative Example 4)

A water-absorbent sheet composition was obtained in the same manner as in Example 3, except that a water-absorbent resin B in Example 3 was changed to a water-absorbent resin E having a irregular and pulverized shape (median particle size: 610 μm; water-absorption rate of saline solution: 77 s; initial water-absorption rate: 0.37 mL/s; effective amount of water absorbed: 44 mL/g). The resulting water-absorbent sheet composition was cut into a given size to measure the properties of the water-absorbent sheet. The results are shown in Table 3.

(Comparative Example 5)

A water-absorbent sheet composition was obtained in the same manner as in Example 5, except that amounts of a water-absorbent resin A and a water-absorbent resin C used were changed as listed in Table 2, that spreading was carried out without adding an adhesive, that a breathabLe fractionating layer was changed to a hydrophilically treated PE-PP nonwoven fabric (basis weight: 22 g/m$^2$), and that a heating temperature of a thermal laminating machine was changed to 80°C, in Example 5. The resulting water-absorbent sheet composition was cut into a given size to measure the properties of the water-absorbent sheet. The results are shown in Table 3.

(Comparative Example 6)

A water-absorbent sheet composition was obtained in the same manner as in Example 5, except that amounts of a water-absorbent resin A, a water-absorbent resin C, and an adhesive used were changed as listed in Table 2, and that a breathable fractionating layer was changed to a hydrophilic nonwoven fabric made of rayon (basis weight: 20g/m$^2$, rayon content: 100%), in Example 5. The resulting water-absorbent sheet composition was cut into a given size to measure the properties of the water-absorbent sheet. The results are shown in Table 3.

[Table 2]

Table 2

| | Hydrophilic Nonwoven Fabric | | Water-Absorbent Resin | | | | | | | | | Adhesive (g/m²) | | | |
|---|---|---|---|---|---|---|---|---|---|---|---|---|---|---|---|
| | | | Primary Absorbent Layer | | | | | Secondary Absorbent Layer | | | | | | | |
| | Top | Bottom | g/m² | Median Particle Size (μm) | Water-Absorption Rate (s) | Initial Water-Absorption Rate (mL/s) | Effective Amount of Water Absorbed (mL/g) | g/m² | Median Particle Size (μm) | Water-Absorption Rate (s) | Ratio * | Kind | Primary | Secondary | Content** |
| Ex. 1 | Rayon | Rayon | 270 | 340 | 38 | 0.17 | 56 | 65 | 240 | 3 | 81/19 | Methylene-vinyl acetate | 55 | 13 | 0.20 |
| Ex. 2 | Rayon | Rayon | 270 | 340 | 38 | 0.17 | 56 | 30 | 240 | 3 | 90/10 | Ethylene-vinyl acetate | 55 | 6 | 0.20 |
| Ex. 3 | Rayon-PET | Rayon-PEF | 270 | 390 | 53 | 0.23 | 63 | 65 | 240 | 3 | 81/19 | Polyester | 55 | 13 | 0.20 |
| Ex. 4 | Rayon-PET | Rayon-per | 270 | 390 | 53 | 0.23 | 63 | 30 | 240 | 3 | 90/10 | Polyester | 55 | 13 | 0.20 |
| Ex. 5 | Rayon-PET | Rayon-pent | 400 | 340 | 38 | 0.17 | 56 | 65 | 240 | 3 | 86/14 | Polyethylene | 100 | 16 | 0.25 |
| Comp. Ex. 1 | Rayon | Rayon | 340 | 340 | 38 | 0.17 | 56 | - | - | - | - | Ethylene-vinyl acetate | 55 | - | 0.16 |
| Comp. Ex. 2 | Rayon | Rayon | (270) | 340 | 38 | 0.17 | 56 | (65) | 240 | 3 | (Mixed) | Ethylene-vinyl acetate | (68) | - | 0.20 |

| | Hydrophilic Nonwoven Fabric | | Water-Absorbent Resin | | | | | | | | | Adhesive (g/m²) | | | |
| | Top | Bottom | Primary Absorbent Layer | | | | | Secondary Absorbent Layer | | | | | | | |
| | | | g/m² | Median Particle Size (μm) | Water-Absorption Rate (s) | Initial Water-Absorption Rate (mL/s) | Effective Amount of Water Absorbed (mL/g) | g/m² | Median Particle Size (μm) | Water-Absorption Rate (s) | Ratio * | Kind | Primary | Secondary | Content** |
| Comp. Ex. 3 | Rayon-PET | Rayon-PET | 270 | 390 | 53 | 0.23 | 63 | 65 | 90 | 7 | 81/19 | Polyester | 55 | 13 | 0.20 |
| Comp. Ex. 4 | Rayon-PET | Rayon-PET | 270 | 610 | 77 | 0.37 | 44 | 65 | 240 | 3 | 81/19 | Polyester | 55 | 13 | 0.20 |
| Comp. Ex. 5 | Rayon-PET | Rayon-PET | 150 | 340 | 38 | 0.17 | 56 | 50 | 240 | 3 | 75/25 | - | - | - | - |
| Comp. Ex. 6 | Rayon-PET | Rayon-PET | 20 | 340 | 38 | 0.17 | 56 | 40 | 240 | 3 | 33/67 | Polyethylene | 5 | 10 | 0.25 |

*: Ratio of primary absorbent layer to secondary absorbent layer, i.e. primary/secondary, of water-absorbent resin (mass ratio)
**: Content of adhesive (content based on water-absorbent resin (mass basis))

[Table 3] Table 3

| | Thickness (mm) | Permeation Rate (s) | | | | Amount of Re-wet (g) | Leakage Test at Inclination | | | | Evaluations of Water-Absorbent Sheet | | |
|---|---|---|---|---|---|---|---|---|---|---|---|---|---|
| | | 1 | 2 | 3 | Total | | 1 | 2 | 3 | Index | Strength | Feel | Operability |
| Ex. 1 | 1.4 | 40 | 28 | 30 | 98 | 3.8 | 0 | 0 | 0 | 0 | ○ | 4.2 | 4.8 |
| Ex. 2 | 1.3 | 38 | 25 | 28 | 91 | 3.5 | 0 | 0 | 0 | 0 | ○ | 4.4 | 4.8 |
| Ex. 3 | 1.3 | 39 | 27 | 28 | 94 | 2.9 | 0 | 0 | 0 | 0 | ○ | 4.0 | 4.8 |
| Ex. 4 | 1.4 | 27 | 20 | 25 | 72 | 2.2 | 0 | 0 | 0 | 0 | ○ | 4.2 | 4.8 |
| Ex. 5 | 1.9 | 42 | 27 | 29 | 98 | 1.6 | 0 | 0 | 0 | 0 | ○ | 4.0 | 4.6 |
| Comp. Ex. 1 | 1.2 | 44 | 30 | 38 | 112 | 3.6 | 25 | 0 | 0 | 250 | △ | 3.8 | 4.8 |
| Comp. Fez. 2 | 1.1 | 46 | 36 | 39 | 121 | 8.6 | 15 | 8 | 13 | 203 | △ | 3.8 | 4.3 |
| Comp. Ex. 3 | 1.2 | 47 | 47 | 57 | 151 | 3.8 | 24 | 2 | 1 | 251 | × | 2.4 | 1.9 |
| Comp. Ex. 4 | 1.4 | 48 | 35 | 42 | 125 | 10.3 | 18 | 4 | 2 | 202 | × | 1.8 | 3.2 |
| Comp. Ex. 5 | 4.5 | 35 | 24 | 44 | 103 | 9.4 | 28 | ×* | - | 280 (for Ref.) | × | 3.8 | 4.6 |
| Comp. Ex. 6 | 1.6 | 49 | 45 | 51 | 145 | 40.2 | 52 | 40 | 38 | 758 | ○ | 3.0 | 4.8 |

×*...A large amount of the water-absorbent resin spilled upon supplying the liquid, thereby breaking down the sheet.

It can be seen from Tables 2 and 3 that as shown in Examples 1 to 6, a water-absorbent sheet composition having a structure in which a water-absorbent resin within the range of the present invention is used in a primary absorbent layer and a secondary absorbent layer, in which each of the absorbent layers are not substantially mixed, or fractionated has a more excellent properties in permeation rates, re-wet, and a leakage index at slope.

On the other hand, when Comparative Examples are studied, in a case where an absorbent layer is a single layer (Comparative Examples 1 and 2), both the evaluations for a total permeation rate and a leakage index are low, thereby making them disadvantageous in the properties of the water-absorbent sheet composition. Even when two kinds of water-absorbent resins are used, as in Comparative Example 2, if an absorbent layer has a single layer structure, improvements in the properties of the water-absorbent sheet composition were not found. In a case where a median particle size or the like of a water-absorbent resin used in a primary absorbent layer is outside the range of the present invention (Comparative Example 4), and a case where a median particle size or the like of a water-absorbent resin used in a secondary absorbent layer is outside the range of the present invention (Comparative Example 3), even if the absorbent layer has a two-layer structure, not only the evaluations of both a total permeation rate and a leakage index were low, but also the strength of a water-absorbent sheet composition was disadvantageous. Further, in a case where two absorbent layers are formed without using an adhesive (Comparative Example 5), a large amount of a water-absorbent resin is spilled upon a slope leakage test, thereby breaking down a water-absorbent sheet composition, and moreover is disadvantageous in strength of a water-absorbent sheet composition, so that these comparative products hardly were provided with fundamental properties for a water-absorbent sheet composition. Furthermore, in a case where an amount of a water-absorbent resin used is small and is outside the range of the present invention (Comparative Example 6), a water-absorbent sheet composition was clearly disadvantageous in the viewpoint of permeation rates, re-wet, and a leakage index, while the strength of a water-absorbent sheet composition can be satisfied, so that the water-absorbent sheet composition was not provided with fundamental properties for a water-absorbent sheet composition.

(Example 7, Comparative Example 7)

Manufacture of Absorbent Articles

A back sheet side of a product manufactured by Procter and Gamble under the trade name Pampers Cottoncare (L size) was cut to open, and the contents were carefully removed so as not to destroy the top sheet. Each of the water-absorbent sheet compositions obtained in Example 2 and Comparative Example 2 cut into pieces of 10 cm × 40 cm were inserted from the cut in a manner that a primary absorbent layer was located on the top sheet side, and sealed, to give absorbent articles (Example 7, Comparative Example 7). A test was conducted on 10 panelists using these absorbent articles. As a result, an evaluation was obtained that the absorbent article of Example 6 is more excellent in the viewpoint of feel, dry feel upon exchanging diapers, and liquid leakage.

INDUSTRIAL APPLICABILITY

The water-absorbent sheet composition of the present invention can be used for absorbent articles in hygienic material fields, agricultural fields, construction material fields, and the like, among which the water-absorbent sheet composition can be suitably used for absorbent articles in the hygienic material fields.

EXPLANATION OF NUMERICAL SYMBOLS

1    burette section
2    lead tube
3    measuring platform
4    nonwoven fabric
5    water-absorbent resin
6    stand
7    clamp
10   burette
11   air inlet tube
12   cock
13   cock
14   rubber plug

21    acrylic plate
22    acrylic plate
23    water-absorbent sheet composition
31    stand
32    acrylic plate
33    water-absorbent sheet composition
34    dropping funnel
35    balance
36    metallic tray
51    water-absorbent sheet composition
52    water-absorbent resin
53    primary absorbent layer
54    water-absorbent resin
55    secondary absorbent layer
57    hydrophilic nonwoven fabric


**Claims**

1.  A water-absorbent sheet composition comprising a structure in which an absorbent layer comprising a water-absorbent resin and an adhesive is sandwiched with two or more sheets of hydrophilic nonwoven fabrics, wherein the water-absorbent sheet composition has a structure in which the absorbent layer is fractionated into a primary absorbent layer and a secondary absorbent layer, and wherein the water-absorbent sheet composition satisfies:

    (1) the median particle size of the water-absorbent resin used in the primary absorbent layer and the median particle size of the water-absorbent resin used in the secondary absorbent layer are from 100 to 600 $\mu$m;
    (2) the water absorption rate of saline solution of the water-absorbent resin used in the primary absorbent layer is from 20 to 70 seconds;
    (3) the water absorption rate of saline solution of the water-absorbent resin used in the secondary absorbent layer is from 1 to 20 seconds; and
    (4) (the water absorption rate of saline solution of the water-absorbent resin used in the primary absorbent layer)-( the water absorption rate of saline solution of the water-absorbent resin used in the secondary absorbent layer) is 10 seconds or more.

2.  The water-absorbent sheet composition according to claim 1, wherein the water-absorbent resin used in the primary absorbent layer has an initial water-absorption rate of 0.35 mL/s or less, and the water-absorbent resin has an effective amount of water absorbed of saline solution of 45 mL/g or more.

3.  The water-absorbent sheet composition according to claim 1 or 2, wherein the water-absorbent resin used in the secondary absorbent layer is a water-absorbent resin obtained by reversed phase suspension polymerization method.

4.  The water-absorbent sheet composition according to any of claims 1 to 3, wherein the water-absorbent resin is contained in an amount of from 100 to 1,000 g/m$^2$, and the adhesive is contained in an amount (mass basis) of from 0.05 to 2,0 times that of the water-absorbent resin.

5.  The water-absorbent sheet composition according to any of claims 1 to 3, wherein the water-absorbent resin is contained in an amount of from 200 to 800 g/m$^2$, and the adhesive is contained in an amount (mass basis) of from 0.05 to 2.0 times that of the water-absorbent resin.

6.  The water-absorbent sheet composition according to any one of claims 1 to 5, wherein the hydrophilic nonwoven fabric is made of at least one member selected from the group consisting of rayon fibers, polyolefin fibers, polyester fibers and mixtures thereof.

7.  The water-absorbent sheet composition according to any one of claims 1 to 6, wherein the adhesive is at least one member selected from the group consisting of polyolefin-based adhesives, polyester-based adhesives, ethylene-vinyl acetate copolymer adhesives and styrenic elastomer adhesives.

8. The water-absorbent sheet composition according to any one of claims 1 to 7, wherein the water-absorbent sheet composition satisfying all the properties of the following (A) to (C):

   (A) the water-absorbent sheet composition having a thickness of 5 mm or less,
   (B) a total permeation rate of 120 seconds or less, and
   (C) a leakage index of 100 or less.

9. An absorbent article comprising the water-absorbent sheet composition as defined in any one of claims 1 to 8, sandwiched between a liquid-permeable sheet and a liquid-impermeable sheet.

[Figure 1]

# FIG. 1

[Figure 2]

FIG. 2

[Figure 3]

FIG. 3

[Figure 4]

FIG. 4

[Figure 5]

15kV   X50   500μm

FIG. 5 (Production Example 1)

[Figure 6]

15kV    X50    500μm

# FIG. 6 (Production Example 3)

[Figure 7]

15kV    X50    500μm

# FIG. 7
# (Commercially Available Product)

[Figure 8]

15kV    X50    500μm

# FIG. 8
# (Commercially Available Product)

**EP 2 387 981 A1**

<table>
<tr><td colspan="2" align="center">**INTERNATIONAL SEARCH REPORT**</td><td colspan="2">International application No.<br>PCT/JP2009/061815</td></tr>
</table>

**A. CLASSIFICATION OF SUBJECT MATTER**
A61F13/49(2006.01)i, A61F13/15(2006.01)i, A61F13/53(2006.01)i, B32B5/26
(2006.01)i, B32B27/00(2006.01)i, B32B27/12(2006.01)i

According to International Patent Classification (IPC) or to both national classification and IPC

**B. FIELDS SEARCHED**

Minimum documentation searched (classification system followed by classification symbols)
A61F13/49, A61F13/15, A61F13/53, B32B5/26, B32B27/00, B32B27/12

Documentation searched other than minimum documentation to the extent that such documents are included in the fields searched
Jitsuyo Shinan Koho          1922–1996   Jitsuyo Shinan Toroku Koho   1996–2009
Kokai Jitsuyo Shinan Koho    1971–2009   Toroku Jitsuyo Shinan Koho   1994–2009

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | CD-ROM of the specification and drawings annexed to the request of Japanese Utility Model Application No. 298/1993(Laid-open No. 59039/1994) (Sekisui Plastics Co., Ltd.), 16 August, 1994 (16.08.94), Full text; all drawings (Family: none) | 1-9 |
| A | CD-ROM of the specification and drawings annexed to the request of Japanese Utility Model Application No. 98906/1991(Laid-open No. 48923/1993) (Sekisui Plastics Co., Ltd.), 29 June, 1993 (29.06.93), Full text; all drawings (Family: none) | 1-9 |

☒ Further documents are listed in the continuation of Box C.       ☐ See patent family annex.

| | |
|---|---|
| * Special categories of cited documents:<br>"A" document defining the general state of the art which is not considered   to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search<br>    16 September, 2009 (16.09.09) | Date of mailing of the international search report<br>    06 October, 2009 (06.10.09) |
|---|---|
| Name and mailing address of the ISA/<br>    Japanese Patent Office | Authorized officer |
| Facsimile No. | Telephone No. |

Form PCT/ISA/210 (second sheet) (April 2007)

**INTERNATIONAL SEARCH REPORT**

| | International application No. |
|---|---|
| | PCT/JP2009/061815 |

C (Continuation).    DOCUMENTS CONSIDERED TO BE RELEVANT

| Category* | Citation of document, with indication, where appropriate, of the relevant passages | Relevant to claim No. |
|---|---|---|
| A | CD-ROM of the specification and drawings annexed to the request of Japanese Utility Model Application No. 299/1993(Laid-open No. 58931/1994) (Sekisui Plastics Co., Ltd.), 16 August, 1994 (16.08.94), Full text; all drawings (Family: none) | 1-9 |
| A | JP 10-118117 A  (Kao Corp.), 12 May, 1998 (12.05.98), Full text; all drawings (Family: none) | 1-9 |

Form PCT/ISA/210 (continuation of second sheet) (April 2007)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP HEI9510889 B
- JP HEI857311 B
- JP 2000238161 A
- JP 2003011118 A
- JP HEI02048944 B
- JP HEI6059039 B